# EUROPÄISCHE PATENTSCHRIFT

(11) **EP 3 359 953 B1**
(45) Veröffentlichungstag und Bekanntmachung des Hinweises auf die Patenterteilung: **15.07.2020**
(21) Anmeldenummer: 16778352.1
(22) Anmeldetag: 06.10.2016
(51) Int. Cl.: G01N 21/64, G01N 33/38, G01N 21/94

(54) **VORRICHTUNG UND VERFAHREN ZUR SCHIMMELDETEKTION AN BAUSTOFFOBERFLÄCHEN**
APPARATUS AND METHOD FOR DETECTION OF MOULD AT THE SURFACE OF BUILDING MATERIALS
DISPOSITIF ET PROCÉDÉ POUR LA DÉTECTION DE MOISISSURE SUR LES SURFACES DES MATÉRIAUX DE CONSTRUCTION

(30) Priorität: 08.10.2015 DE 102015219540
(43) Veröffentlichungstag der Anmeldung: 15.08.2018
(73) Patentinhaber: Fraunhofer-Gesellschaft zur Förderung der angewandten Forschung e.V., 80686 München (DE)
(72) Erfinder: SCHULTE, Henning, 96450 Coburg (DE); GRUNA, Robin, 76530 Baden-Baden (DE); HOFBAUER, Wolfgang Karl, 6330 Kufstein (AT)
(74) Vertreter: Schenk, Markus
(86) Internationale Anmeldenummer: PCT/EP2016/073911
(87) Internationale Veröffentlichungsnummer: WO 2017/060373

(56) Entgegenhaltungen:
- DE-A1- 10 100 581
- DE-A1-102005 051 643
- Anonymous: "Sicher mit Fluoreszenz", Sicher mit Fluoreszenz, 1. März 2010 (2010-03-01), Seiten 1-1, XP055323786, Gefunden im Internet: URL:http://www.hund.de/images/Publikatione n/exakt_Mrz_2010_S._17_Sicherer_mit_Fluore szenz.pdf [gefunden am 2016-11-28] & Anonymous: "Direktnachweis von Mykosen-Fluoreszenz statt Hellfeld", Galabau, 4. April 2011 (2011-04-04), Seiten 1-1, XP055324087, Gefunden im Internet: URL:http://www.hund.de/images/Publikatione n/www.soll-galabau.de_Direktnachweis_von_M ykosen-Fluoreszenz_statt_Hellfeld_Artikel_ vom_04.04.2011.pdf [gefunden am 2016-11-29]
- ERIC SAVORY ET AL: "An optoelectronic sensor for the monitoring of mould growth in concealed spaces", BUILDING AND ENVIRONMENT, PERGAMON PRESS, OXFORD, GB, Bd. 49, 20. September 2011 (2011-09-20), Seiten 9-16, XP028113622, ISSN: 0360-1323, DOI: 10.1016/J.BUILDENV.2011.09.023 [gefunden am 2011-09-28]
- M.-C. BÉLANGER ET AL: "Autofluorescence of grape berries following Botrytis cinerea infection", INTERNATIONAL JOURNAL OF REMOTE SENSING, Bd. 32, Nr. 14, 20. Juli 2011 (2011-07-20) , Seiten 3835-3849, XP055324429, GB ISSN: 0143-1161, DOI: 10.1080/01431161003782064
- Kristian D Nielsen: "Mould growth on building materials", , 1. Januar 2002 (2002-01-01), Seiten 1-122, XP055323883, DK-2970 Hørsholm ISBN: 978-87-5631-130-4 Gefunden im Internet: URL:http://www.sbi.dk/indeklima/fugt-skimm el/mould-growth-on-building-materials/2006 -01-12.6153316360/at_download/file [gefunden am 2016-11-29]

## Beschreibung

Die Erfindung betrifft eine Vorrichtung zum Erfassen und Bestimmen einer Menge von unter Bestrahlung mit ultraviolettem Licht fluoreszierendem Schimmel auf Baustoffoberflächen mit den Merkmalen von Anspruch 1, sowie ein entsprechendes Verfahren mit den Merkmalen von Anspruch 10.

Die Kontamination von Baustoffoberflächen mit Schimmel ist ein seit langem bestehendes und bekanntes Problem. Schimmel entsteht vorwiegend an feuchten Stellen. Das Auftreten von Schimmel an Baustoffoberflächen kann somit ein Hinweis darauf sein, dass an der befallenen Stelle eine zu hohe Feuchtigkeit vorherrscht. Schimmel ist darüber hinaus potentiell gesundheitsgefährdend für Menschen.

Die Kontamination von Baustoffoberflächen durch Schimmel wird heute meist durch den Ansatz von Kulturen aus der jeweiligen Verdachtsstelle ermittelt. Hierbei werden Schimmelsporen oder Schimmelkulturen gesammelt und auf einem Nährmedium zur Beobachtung des Wachstums im Labor gezüchtet. Das "Sammeln" der Schimmelkulturen bzw. Schimmelsporen geschieht dabei in der Regel entweder durch Filtration aus der Raum- bzw. Umgebungsluft oder durch Abtragen direkt von der vermeintlich kontaminierten Fläche oder durch Aufarbeiten von Materialproben.

Derartige Verfahren zum Nachweis von Schimmel sind beispielsweise aus der DE 690 07 482 T2 sowie aus der DE 10 2004 031 197 A1 bekannt. In beiden Veröffentlichungen werden Proben genommen, die dann in einem Labor untersucht werden. Die DE 690 07 482 T2 beschreibt ein gattungsgemäßes Verfahren, das sich ebenfalls mit Schimmel an Baumaterialien befasst. Hierbei geht es darum, die Lebensfähigkeit von Pilzzellen nach einer Zell-Abtötungsbehandlung zu ermitteln. Nach der Zell-Abtötungsbehandlung vor Ort wird von dem befallenen Baumaterial eine Probe des Pilzes genommen. Diese Probe wird zunächst einige Zeit abgelagert, dann mit Fluoreszenzfarbstoffen eingefärbt und fixiert. Die weitere Untersuchung des Pilzes, d.h. ob dieser vollständig abgestorben ist, erfolgt abseits der Probeentnahmestelle in einem Labor.

Diese bekannten Methoden sind zwar gut geeignet, um Schimmelbefall qualitativ nachzuweisen und die Schimmelart zu bestimmen. Die Methoden sind jedoch zeitaufwändig, da das Heranzüchten von Schimmelkulturen auf Nährmedien mehrere Tage bis Wochen dauern kann. Außerdem wird die zu untersuchende Baustoffoberfläche durch das Abtragen des Schimmels meist zerstört.

Es sind außerdem Verfahren bekannt, bei denen Schimmelsporen in der Raumluft in Echtzeit gezählt werden. Derartige Echtzeitverfahren funktionieren zwar zur Bestimmung in der Raumluft, sie erfassen den Schimmel jedoch nur unzureichend qualitativ. Hierbei wird außerdem keine Bestimmung der Schimmelsporen auf einer Baustoffoberfläche durchgeführt.

Es ist daher eine Aufgabe der vorliegenden Erfindung, ein Verfahren sowie eine Vorrichtung bereitzustellen, die es ermöglichen, einen schnellen sowohl qualitativen als auch quantitativen Nachweis von Schimmel zu erbringen, wobei die Vorrichtung vor Ort einsetzbar ist und vorzugsweise zerstörungs- und beschädigungsfrei an einer Baustoffoberfläche nutzbar ist.

Diese Aufgabe wird erfindungsgemäß mit einer Vorrichtung mit den Merkmalen gemäß Anspruch 1, sowie mit einem Verfahren mit den Merkmalen gemäß Anspruch 10 gelöst.

Mit der erfindungsgemäßen Vorrichtung kann ein zu untersuchender Bereich einer Baustoffoberfläche qualitativ auf Vorhandensein von Schimmel untersucht werden. Basierend auf dieser qualitativen Bestimmung von Schimmel in einem zu untersuchenden Bereich kann die erfindungsgemäße Vorrichtung eine quantitative Bestimmung von Schimmel auf zumindest einem Teil der Baustoffoberfläche ermöglichen. Zu der Baustoffoberfläche zählt auch der oberflächennahe Porenraum des Baustoffs. Die erfindungsgemäße Vorrichtung ermöglicht es, Schimmel mit fluoreszierenden Eigenschaften zu erfassen und die Menge an Schimmel quantitativ zu bestimmen. Die erste der beiden Lichtquellen sendet Licht mit einem ultravioletten (UV) Anteil, oder reines UV-Licht, aus. Eine zweite Lichtquelle ist ausgebildet, um Licht in einem anderen Wellenlängenbereich im Vergleich zu der ersten Lichtquelle zu emittieren. Manche Schimmelarten werden von diesem UV-Licht zur Fluoreszenz angeregt. Derartige Schimmelarten senden bei Bestrahlung mit UV-Licht also fluoreszierendes Licht aus. Der Lichtempfänger empfängt einerseits diese von dem Schimmel ausgesandten Fluoreszenzlichtanteile sowie gegebenenfalls weitere von der Baustoffoberfläche reflektierte Lichtanteile und macht Aufnahmen von Bereichen, die jeweils mit der ersten und der zweiten Lichtquelle beleuchtet werden. Die Steuereinheit erkennt die spezifische Reflexion von Schimmel. Diese Reflexion kann auch Fluoreszenzanteile beinhalten. Die Steuereinheit ist in der Lage, diese spezifische Reflexion und darin vorkommende Fluoreszenzlichtanteile in einem Vergleich der Aufnahmen zu erkennen. Falls die Steuereinheit also Fluoreszenzlichtanteile erkannt hat, weiß die Steuereinheit, dass auf dem zu untersuchenden Bereich der Baustoffoberfläche mit großer Wahrscheinlichkeit Schimmel mit fluoreszierenden Eigenschaften vorhanden ist. Dies entspricht der qualitativen Erfassung von Schimmel in dem zu untersuchenden Bereich. Basierend auf dieser ausgeführten qualitativen Erfassung von Schimmel kann die Steuereinheit die Schimmelmenge auf zumindest einem Teil der Baustoffoberfläche quantitativ bestimmen. Hierfür kann die Steuereinheit beispielsweise den zu untersuchenden Bereich stichprobenartig überprüfen. Es muss nicht der gesamte zu untersuchende Bereich überprüft werden. Es kann ausreichend sein, lediglich einen Abschnitt bzw. einen Teilbereich des zu untersuchenden Bereichs zu überprüfen. In beiden Fällen wird zumindest ein Teil der Baustoffoberfläche überprüft. Bei einer stichprobenartigen Überprüfung können die Ergebnisse der Stichproben mittels bekannter Entwicklungswerte des entdeckten Schimmels extrapoliert und statistisch auf den gesamten zu untersuchenden Bereich hochgerechnet werden. Eine quantitative Bestimmung der Schimmelmenge kann die Bestimmung der Schimmelmenge pro Fläche sein. Dies kann beispielsweise in Anzahl der Sporen pro Fläche, oder in Schimmeldichte pro Fläche, oder in Dicke einer Schimmelschicht pro Fläche, oder in Gewicht bzw. Masse des Schimmels pro Fläche angegeben werden.

Es ist denkbar, dass die Vorrichtung eine spektral auflösende Optik aufweist, die ausgebildet ist, um das empfangene Licht in sein Spektrum zu zerlegen, und wobei die Steuereinheit ausgebildet ist, um dieses Spektrum dahingehend zu überprüfen, ob das empfangene Licht zumindest anteilig von Schimmel fluoresziertes Licht aufweist. Die spektral auflösende Optik kann beispielsweise ein Spektrometer sein. Mittels der spektral auflösenden Optik wird das von der Baustoffoberfläche empfangene Licht in sein Spektrum zerlegt und ausgewertet. Unter der Auswertung ist die Bestimmung der Intensität über die Wellenlänge von empfangenen Signalen zu verstehen. Das Spektrum kann sich in Abhängigkeit von der Schimmelkontamination, d.h. in Abhängigkeit von der Menge und Art des Schimmels, ändern.

Es ist vorstellbar, dass die spektral auflösende Optik einen Punktsensor aufweist, der ausgebildet ist, um einen Bildpunkt innerhalb des zu untersuchenden Bereichs zu bestimmen und das in diesem Bildpunkt erfasste Licht in sein Spektrum zu zerlegen. Ein solcher Punktsensor bietet einen einfachen Aufbau und eine schnelle Möglichkeit zur Bestimmung der Schimmelmenge. Der zu untersuchende Bereich der Baustoffoberfläche ist in der Regel größer als ein Bildpunkt. Der Punktsensor untersucht somit lediglich einen Ausschnitt des zu untersuchenden Bereichs der Baustoffoberfläche. Der Punktsensor kann allerdings an mehreren Stellen innerhalb des zu untersuchenden Bereichs eingesetzt werden, um so insgesamt eine größere Fläche innerhalb des zu untersuchenden Bereichs zu untersuchen.

In einer alternativen Ausführungsform kann die spektral auflösende Optik einen Flächensensor aufweisen, der ausgebildet ist, um einen Bildbereich mit 2x2 und mehr Bildpunkten innerhalb des zu untersuchenden Bereichs zu bestimmen und das in diesem Bildbereich erfasste Licht in sein Spektrum zu zerlegen. In dieser Ausführungsform deckt die spektral auflösende Optik einen größeren Bildbereich innerhalb des zu untersuchenden Bereichs ab. Der bestimmte Bildbereich kann kleiner oder auch gleich groß sein wie der zu untersuchende Bereich. Somit kann gegenüber dem Punktsensor eine größere Fläche des zu untersuchenden Bereichs untersucht werden.

Es ist vorstellbar, dass die Steuereinheit ausgebildet ist, um eine Absorption und/oder Reflexion bestimmter Wellenlängenbereiche in dem empfangenen Licht des zu untersuchenden Bereichs zu erfassen und diese absorbierten und/oder reflektierten Wellenlängenbereiche als Identifikationsmerkmal zur Bestimmung der Schimmelart heranzuziehen. Unterschiedliche Schimmelarten absorbieren unterschiedliche Wellenlängenbereiche. Mittels der spektralen Optik können die verschiedenen, für unterschiedliche Schimmelarten charakteristischen, Wellenlängenbereiche detektiert werden. Eine automatische Klassifizierungseinheit kann die ermittelten Spektrogramme auswerten und einer bestimmten Schimmelart zuordnen.

Eine beispielhafte, aber nicht beanspruchte Lichtquelle kann eine spektral breitbandige Beleuchtung aufweisen, deren Spektrum den Messbereich der spektral auflösenden Optik überdeckt. Somit kann sichergestellt werden, dass der zu untersuchende Bereich in einem möglichst breitbandigen Spektrum ausgeleuchtet wird. Der Messbereich der spektral auflösenden Optik liegt demnach vollständig innerhalb des Spektrums des von der Lichtquelle ausgesendeten Lichts. Somit kann das gesamte Spektrum mittels der spektral auflösenden Optik vermessen werden.

Gemäß der beanspruchten Erfindung weist die Vorrichtung eine zweite Lichtquelle auf, die Licht in einem anderen Wellenlängenbereich im Vergleich zu der ersten Lichtquelle emittiert, und beide Lichtquellen können sequentiell schaltbar sein. Die erste Lichtquelle emittiert ultraviolettes Licht in einem Wellenlängenbereich, der für das menschliche Auge nicht sichtbar ist. Die zweite Lichtquelle hingegen kann beispielsweise Licht in einem für das menschliche Auge sichtbaren Wellenlängenbereich emittieren. Beide Lichtquellen sind sequentiell schaltbar, d.h. sie können nacheinander ein- und ausgeschaltet werden. Somit kann der zu untersuchende Bereich beispielsweise einmal mit nicht sichtbarem UV-Licht und einmal mit sichtbarem Licht beleuchtet werden.

Mindestens eine der beiden Lichtquellen kann eine homogene Lichtquelle sein. Eine homogene Lichtquelle eignet sich besonders gut zur gleichmäßigen Beleuchtung des zu untersuchenden Bereichs und eine homogene Lichtquelle liefert darüber hinaus ein sehr genaues und reproduzierbares Ergebnis bei der Spektralzerlegung.

Es ist anspruchsgemäß vorgesehen, dass die Steuereinheit ausgebildet ist, um eine Aufnahme des mit der ersten Lichtquelle beleuchteten zu untersuchenden Bereichs mit einer Aufnahme des mit der zweiten Lichtquelle beleuchteten zu untersuchenden Bereichs zu vergleichen. Die beiden Lichtquellen senden Licht in unterschiedlichen Wellenlängenbereichen aus. Somit kann der zu untersuchende Bereich mit Licht unterschiedlicher Wellenlängen beleuchtet werden. Je nach Lichtquelle liefert die Spektralanalyse dementsprechend andere Werte für das jeweils von der Baustoffoberfläche reflektierte Licht. Die unterschiedlichen Spektrogramme können von der Steuereinheit ausgewertet werden. Somit kann eine fehlererkennende Vorrichtung realisiert werden. Beispielsweise kann der zu untersuchende Bereich eine Stelle aufweisen, die unter Bestrahlung der ersten Lichtquelle, z.B. mit UV-Licht, fluoreszierendes Licht aussendet. Dies kann ein Hinweis auf Schimmel sein. Unter Bestrahlung mit der zweiten Lichtquelle kann dieselbe Stelle jedoch z.B. eine Reflexion zeigen, die nicht für Schimmel charakteristisch ist. Somit kann in diesem Fall ausgeschlossen werden, dass an ebendieser Stelle Schimmel vorhanden ist, obwohl diese Stelle bei Beleuchtung mit der ersten Lichtquelle fluoreszierendes Licht ausgesandt hat.

Gemäß einer Ausführungsform kann die Steuereinheit ausgebildet sein, um für eine stichprobenartige Bestimmung der Schimmelmenge eines Teils der Baustoffoberfläche, die Baustoffoberfläche in eine Anzahl zu untersuchender Bereiche zu unterteilen, wobei die Anzahl zu untersuchender Bereiche sowie eine Anzahl in den jeweiligen zu untersuchenden Bereichen durchzuführender Stichproben basierend auf bekannten Hauptansiedlungspunkten des Schimmels und/oder bekannten Ausbreitungscharakteristiken des Schimmels bestimmt wird. Gemäß dieser Ausführungsform wird ein gewisser Teil der Baustoffoberfläche untersucht. Hierfür wird die Baustoffoberfläche, wie zum Beispiel eine Wand, in mindestens einen, vorzugsweise aber mehrere zu untersuchende Bereiche unterteilt. Diese zu untersuchenden Bereiche können hierbei beispielsweise Quadranten bestimmter Größe sein. Innerhalb dieser Quadranten werden Stichproben, z.B. hinsichtlich Auftreten, Art und Menge von Schimmel, durchgeführt. Die Steuereinheit berechnet sowohl die Größe der Quadranten, als auch die Anzahl an Stichproben innerhalb eines Quadranten sowie die genauen Messpunkte für die Stichprobennahme innerhalb eines Quadranten. Grundlage für diese Berechnung sind bekannte Haupt-Kontaminationspunkte innerhalb des Quadranten sowie bekannte Entwicklungsmechanismen der Schimmelkontamination. Die innerhalb eines Quadranten genommenen Stichproben liefern im Ergebnis eine bestimmte Menge bzw. Konzentration an Schimmel. Die Steuereinheit kann dieses Ergebnis auf eine statistische Verteilungswahrscheinlichkeit einer Menge an Schimmel innerhalb dieses gesamten Quadranten hochrechnen.

Gemäß einer weiteren Ausführungsform kann die Steuereinheit ausgebildet sein, um für eine statistische Bestimmung der Schimmelmenge auf der gesamten Baustoffoberfläche die innerhalb der bestimmten Anzahl von zu untersuchenden Bereichen zuvor erfasste Schimmelmenge zu extrapolieren, um die Schimmelmenge statistisch auf die gesamte Baustoffoberfläche hochzurechnen. Gemäß dieser Ausführungsform ist der Steuereinheit aufgrund der zuvor innerhalb des einen oder innerhalb der mehreren Quadranten durchgeführten Bestimmung der Schimmelmenge die Konzentration bzw. Schimmelmenge in ebendiesen untersuchten Bereichen der Baustoffoberfläche bekannt. Diese bekannten Werte kann die Steuereinheit nun extrapolieren, um die Konzentration an Schimmel statistisch auf die gesamte Baustoffoberfläche, wie z.B. auf eine gesamte Wand, hochzurechnen. So kann mittels Stichprobennahme in einem Teil der Baustoffoberfläche auf die Schimmelmenge der gesamten Baustoffoberfläche geschlossen werden.

Ein weiterer Aspekt der Erfindung betrifft ein Verfahren zum Erfassen und Bestimmen einer Menge von unter Bestrahlung mit ultraviolettem Licht fluoreszierendem Schimmel auf Baustoffoberflächen. Das Verfahren beinhaltet die in Anspruch 10 definierten Verfahrensschritte. Es wird also zunächst mindestens ein zu untersuchender Bereich auf der Baustoffoberfläche festgelegt. Bei einer großen Baustoffoberfläche, wie z.B. bei einer Wand, können mehrere zu untersuchende Bereiche festgelegt werden, wobei jeder der zu untersuchenden Bereiche kleiner ist als die Wand selbst. Bei kleinen Baustoffoberflächen, wie z.B. bei einem einzelnen Stein, kann es ausreichend sein, lediglich einen einzigen zu untersuchenden Bereich festzulegen, der möglicher Weise die selbe Größe wie der Stein selbst aufweist. Falls in einem zu untersuchenden Bereich Schimmel vorhanden sein sollte, wird dieser qualitativ erfasst. Mit diesem Verfahren kann insbesondere Schimmel erfasst werden, der die Eigenschaft hat, unter Bestrahlung mit geeignetem Licht (z.B. UV-Licht) zu fluoreszieren. Hierfür wird der mindestens eine zu untersuchende Bereich mit UV-Licht beleuchtet. Es wird überprüft, ob das von dem zu untersuchenden Bereich empfangene Licht gegebenenfalls fluoreszierende Anteile aufweist. Falls Fluoreszenz erkannt wurde, kann dies auf Schimmel mit fluoreszierenden Eigenschaften hindeuten. Dieser Schimmel wird somit also qualitativ erkannt bzw. erfasst. Basierend auf dieser qualitativen Erkennung bzw. Erfassung in dem zu untersuchenden Bereich kann nun die Menge an Schimmel innerhalb des zu untersuchenden Bereichs ermittelt werden. Dies kann durch geeignete, beispielsweise statistische, Auswerteverfahren geschehen. Da der zu untersuchende Bereich, wie eingangs erwähnt, kleiner sein kann, als die Baustoffoberfläche, wird demgemäß die Schimmelmenge auf zumindest einem Teil der Baustoffoberfläche quantitativ bestimmt. Genauer gesagt wird die Schimmelmenge auf zumindest einem Teil der Baustoffoberfläche auf Grundlage der zuvor in zumindest einem zu untersuchenden Bereich der Baustoffoberfläche ausgeführten qualitativen Erfassung von Schimmel quantitativ bestimmt. Dieses Verfahren hat den Vorteil, dass es direkt vor Ort angewandt werden kann. Es kann außerdem direkt an der Baustoffoberfläche ausgeführt werden, ohne die Baustoffoberfläche selbst dabei zerstören zu müssen. Das erfindungsgemäße Verfahren liefert die Ergebnisse, d.h. die quantitative Bestimmung der Schimmelmenge, darüber hinaus innerhalb kürzester Zeit, vorzugsweise in Echtzeit.

Es ist denkbar, dass in dem erfindungsgemäßen Verfahren vor dem Überprüfen, ob das empfangene Licht zumindest anteilig von Schimmel fluoresziertes Licht aufweist, das empfangene Licht in dessen Spektrum zerlegt wird. Eine Spektralzerlegung des von der Baustoffoberfläche bzw. von dem zu untersuchenden Bereich empfangenen Lichts kann die Erkennung von Fluoreszenzanteilen in dem Licht erleichtern.

Gemäß einer Ausführungsform kann das erfindungsgemäße Verfahren weiterhin aufweisen ein Erfassen einer Absorption bestimmter Wellenlängenbereiche in dem empfangenen Licht des zu untersuchenden Bereichs, und ein Heranziehen dieser absorbierten Wellenlängenbereiche als Identifikationsmerkmal zur Bestimmung der Schimmelart. Es gibt Schimmelarten, die neben der mittels UV-Licht angeregten Fluoreszenz Licht unterschiedlicher Wellenlängen reflektieren oder absorbieren. Durch die Erfassung der Absorptions- bzw. Reflexionsspektren in den zu untersuchenden Bereichen kann somit festgestellt werden, um welche Art von Schimmel es sich bei dem detektierten Schimmel handelt. Mit dem erfindungsgemäßen Verfahren kann also eine qualitative Erfassung von Schimmel, die quantitative Bestimmung der Menge an Schimmel und die Art des Schimmels ermittelt werden.

Das Verfahren nach dem unabhängigen Anspruch 10 beinhaltet ein Bereitstellen einer ersten Lichtquelle zum Aussenden des ultravioletten Lichts, ein Bereitstellen einer zweiten Lichtquelle zum Aussenden von Licht in einem im Vergleich zu der ersten Lichtquelle verschiedenen Wellenlängenbereich, und ein sequentielles Schalten der ersten und der zweiten Lichtquelle. Die erste Lichtquelle emittiert ultraviolettes Licht in einem Wellenlängenbereich, der für das menschliche Auge nicht sichtbar ist. Die zweite Lichtquelle hingegen kann beispielsweise Licht in einem für das menschliche Auge sichtbaren Wellenlängenbereich emittieren. Beide Lichtquellen werden sequentiell geschaltet, d.h. sie werden nacheinander ein- und ausgeschaltet. Somit kann der zu untersuchende Bereich mit nicht sichtbarem UV-Licht oder mit sichtbarem Licht beleuchtet werden.

Ausführungsbeispiele der Erfindung sind in der Zeichnung dargestellt und werden nachstehend erläutert. Es zeigen:
- Fig. 1: eine schematische Darstellung einer beispielhaften Vorrichtung zum Erfassen von Schimmel,
- Fig. 2: eine schematische Darstellung einer weiteren beispielhaften Vorrichtung zum Erfassen von Schimmel,
- Fig. 3: eine schematische Darstellung einer weiteren beispielhaften Vorrichtung zum Erfassen von Schimmel,
- Fig. 4A: ein exemplarisches Spektrogramm einer Aufnahme in einem zu untersuchenden Bereich,
- Fig. 4B: ein weiteres exemplarisches Spektrogramm einer Aufnahme in einem zu untersuchenden Bereich,
- Fig. 4C: ein weiteres exemplarisches Spektrogramm einer Aufnahme in einem zu untersuchenden Bereich,
- Fig. 5: eine Ausführungsform einer erfindungsgemäßen Vorrichtung,
- Fig. 6: eine exemplarische Darstellung einer von Schimmel befallenen Baustoffoberfläche,
- Fig. 7: ein Blockdiagramm eines beispielhaften Verfahrens.

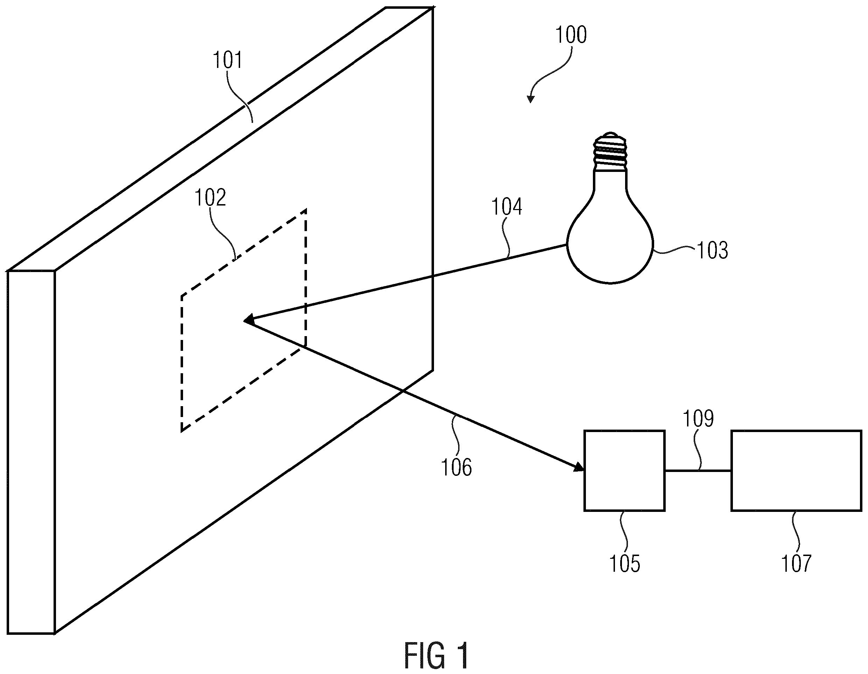

Figur 1 zeigt eine beispielhafte Vorrichtung 100 zum Erfassen und Bestimmen einer Menge von unter Bestrahlung mit ultraviolettem Licht fluoreszierendem Schimmel auf Baustoffoberflächen 101. Die exemplarisch abgebildete Baustoffoberfläche 101 ist eine Wand. Die Wand 101 weist einen zu untersuchenden Bereich 102 auf.

Die Vorrichtung 100 weist eine Lichtquelle 103 auf. Die Lichtquelle 103 ist ausgebildet, um ultraviolettes Licht 104 auf den zu untersuchenden Bereich 102 der Baustoffoberfläche 101 auszusenden. Die Lichtquelle 103 kann entweder rein ultraviolettes Licht 104 oder Licht 104 mit einem Anteil an ultraviolettem Licht aussenden.

Die Vorrichtung 100 weist einen Lichtempfänger 105 auf. Der Lichtempfänger 105 ist ausgebildet, um Licht 106 von dem zu untersuchenden Bereich 102 zu empfangen.

Die Vorrichtung 100 weist eine Steuereinheit 107 auf. Die Steuereinheit 107 ist ausgebildet, um das mit dem Lichtempfänger 105 empfangene Licht 106 dahingehend zu überprüfen, ob das empfangene Licht 106 zumindest anteilig von Schimmel fluoresziertes Licht aufweist. Die Steuereinheit 107 ist ferner ausgebildet, um, in dem Fall, dass ein fluoreszierender Lichtanteil erkannt wurde, die Schimmelmenge auf zumindest einem Teil der Baustoffoberfläche 101, auf Grundlage einer zuvor in mindestens einem zu untersuchenden Bereich 102 der Baustoffoberfläche ausgeführten qualitativen Erfassung von Schimmel, quantitativ zu bestimmen. Die Steuereinheit 107 ist über eine Signalleitung 109 mit der restlichen Vorrichtung 100 verbunden. Die Steuereinheit 107 kann vor Ort an der Vorrichtung 100 vorgesehen sein. Es ist aber auch denkbar, dass die Steuereinrichtung 107 über eine Signalleitung 109, wie beispielsweise einer Netzwerkleitung, von der Vorrichtung 100 entfernt vorgesehen ist. Beispielsweise kann die Steuereinrichtung 107, bzw. die Auswertefunktionalität der Steuereinrichtung 107, auf einem entfernten Rechner, einem Server bzw. in einer Cloud integriert sein. Die Signalleitung 109 kann eine drahtgebundene oder eine drahtlose Signalverbindung sein. Die Signalverbindung kann per Hardware, z.B. mittels einem Kabel, oder kabellos, z.B. mittels W-LAN, Bluetooth, oder anderen Funkstandards, erfolgen. Weiter können Steuereinheit 107 und Lichtempfänger 105 auch in einer gemeinsamen Einheit, z.B. auf einer Platine, aufgebaut sein. Die Steuereinheit 107 kann real, z.B. als ein eigener Rechner, oder virtuell, z.B. in einer Cloud, sein.

Die Lichtquelle 103 kann beispielsweise eine LED sein. Die Lichtquelle 103 sendet ultraviolettes Licht 104 aus. Die Lichtquelle 103 kann entweder ausgebildet sein, um nur ultraviolettes Licht 104 auszustrahlen, oder um Licht 104 mit einem breiteren Spektrum und einem UV-Anteil auszustrahlen.

Der Lichtempfänger 105 kann beispielsweise eine Fotodiode sein. Der Lichtempfänger 105 ist ausgebildet, um zumindest Licht 106 im UV-Wellenlängenbereich zu empfangen. Der Lichtempfänger 105 kann aber auch ausgebildet sein, um Licht 106 mit einem breiteren Spektrum und einem UV-Anteil zu empfangen.

Das Licht 104, welches zumindest einen UV-Anteil aufweist, wird von der Lichtquelle 103 auf den zu untersuchenden Bereich 102 der Baustoffoberfläche 101 ausgestrahlt. Das Licht 104 wird von dem zu untersuchenden Bereich 102 teilweise absorbiert und teilweise reflektiert. In dem zu untersuchenden Bereich 102 kann sich Schimmel, der fluoreszierende Eigenschaften aufweist, befinden. Dieser Schimmel wird dann durch die mittels UV-Licht induzierte Anregung fluoreszieren. Das fluoreszierte Licht wird von dem auf dem zu untersuchenden Bereich 102 befindlichen Schimmel ausgestrahlt. Das Licht 106, das von dem zu untersuchenden Bereich 102 ausgeht, kann somit also sowohl einen reflektiven Anteil, der von der Baustoffoberfläche 101 reflektiert wird, als auch einen fluoreszierenden Anteil, der von Schimmel ausgesandt wird, aufweisen.

Das von dem zu untersuchenden Bereich 102 ausgehende Licht 106 wird von dem Lichtempfänger 105 empfangen. Der Lichtempfänger 105 ist zumindest für die Wellenlängenbereiche empfindlich, die dem von dem Schimmel ausgehenden Fluoreszenz-Lichtanteil entsprechen.

Die Steuereinheit 107 ist mittels der Signalleitung 109 mit dem Lichtempfänger 105 verbunden. Die Steuereinheit 107 wertet das von dem Lichtempfänger 105 empfangene Licht 106 aus. Die Steuereinheit 107 überprüft, ob das empfangene Licht 106 Anteile aufweist, die auf Schimmel schließen lassen könnten. Genauer gesagt überprüft die Steuereinheit 107, ob in dem empfangenen Licht 106 Lichtanteile vorhanden sind, die von Fluoreszenz herrühren. Falls Fluoreszenz-Lichtanteile erkannt wurden, kann dies ein Anzeichen für Schimmel sein. Die Steuereinheit 107 deutet das Vorhandensein des Fluoreszenz-Lichtanteils als Vorhandensein von Schimmel. Die Steuereinheit 107 erfasst somit also qualitativ das Vorhandensein von Schimmel in dem untersuchten Bereich 102 der Baustoffoberfläche 101.

Basierend auf dieser qualitativen Erfassung von Schimmel in dem zu untersuchenden Bereich 102 bestimmt die Steuereinheit 107 anschließend quantitativ die Schimmelmenge in zumindest einem Teil der Baustoffoberfläche 101. Hierfür kann die Steuereinheit 107 beispielsweise quantitativ die Schimmelmenge in dem zu untersuchenden Bereich 102 bestimmen. Die Steuereinheit 107 kann aber auch die Schimmelmenge in lediglich einem Teilbereich des zu untersuchenden Bereichs 102 bestimmen, wobei im Extremfall lediglich ein Punkt aus dem zu untersuchenden Bereich 102 bestimmt wird. Da der zu untersuchende Bereich 102 in dem in Figur 1 gezeigten Beispiel kleiner ist als die gesamte Baustoffoberfläche 101, bestimmt die Steuereinheit 107 somit also quantitativ die Schimmelmenge auf zumindest einem Teil der Baustoffoberfläche 101, d.h. auf dem Teil der Baustoffoberfläche 101, der hier beispielhaft der Größe des zu untersuchenden Bereichs 102 entspricht.

Die Steuereinheit 107 kann aber auch lediglich einen Ausschnitt bzw. ein Teilstück des zu untersuchenden Bereichs 102 auf Vorhandensein und Quantität von Schimmel überprüfen. In diesem Fall bestimmt die Steuereinheit 107 also quantitativ die Schimmelmenge auf zumindest einem Teil der Baustoffoberfläche 101, d.h. auf dem Teil der Baustoffoberfläche 101, der hier der Größe des untersuchten Teilstücks (im Extremfall nur ein Punkt des zu untersuchenden Bereichs 102) des zu untersuchenden Bereichs 102 entspricht.

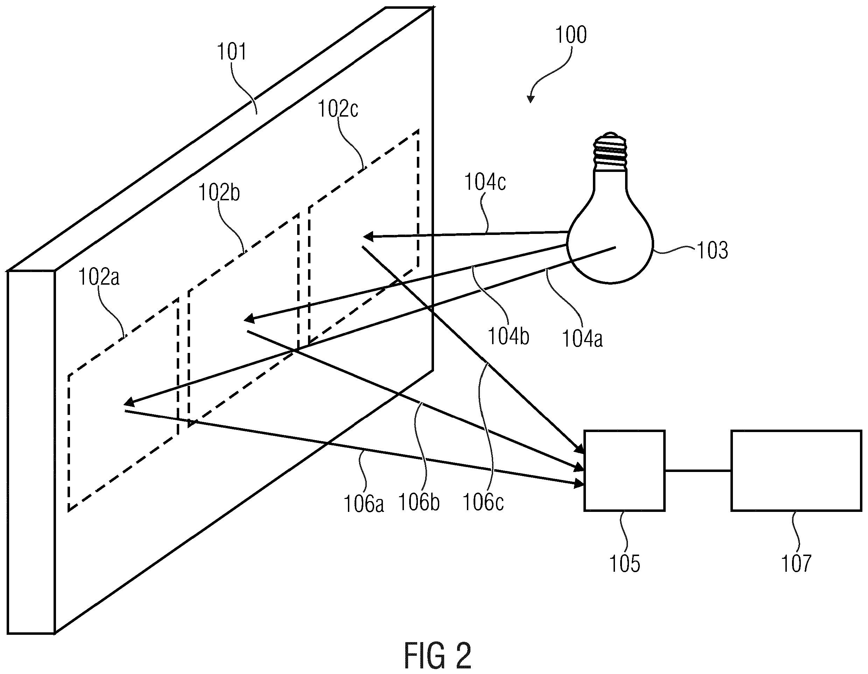

Wie in Figur 2 gezeigt ist, kann die Baustoffoberfläche 101 auch mehrere zu untersuchende Bereiche 102a, 102b, 102c aufweisen. Dieses Beispiel entspricht dem zuvor mit Bezug auf Figur 1 beschriebenen Beispiel, mit dem Unterschied, dass die Baustoffoberfläche 101 einen ersten, zweiten und dritten zu untersuchenden Bereich 102a, 102b, 102c aufweist. In anderen Worten wird die Baustoffoberfläche 101 in mehrere bzw. entsprechend viele zu untersuchende Bereiche 102a, 102b, 102c aufgeteilt.

Die Lichtquelle 103 sendet UV-Licht bzw. breitbandigeres Licht mit einem UV-Anteil aus, wobei ein erster UV-Licht aufweisender Lichtstrahl 104a auf den ersten zu untersuchenden Bereich 102a, ein zweiter UV-Licht aufweisender Lichtstrahl 104b auf den zweiten zu untersuchenden Bereich 102b, und ein dritter UV-Licht aufweisender Lichtstrahl 104c auf den dritten zu untersuchenden Bereich 102c trifft.

Der Lichtempfänger 105 empfängt einen von dem ersten zu untersuchenden Bereich 102a ausgehenden Lichtstrahl 106a, einen von dem zweiten zu untersuchenden Bereich 102b ausgehenden Lichtstrahl 106b sowie einen von dem dritten zu untersuchenden Bereich 102c ausgehenden Lichtstrahl 106c.

Die Steuereinheit 107 überprüft, ob ein Fluoreszenz-Lichtanteil in den empfangenen Lichtstrahlen 106a, 106b, 106c vorhanden ist. Falls ein Fluoreszenz-Lichtanteil festgestellt werden konnte, schließt die Steuereinheit 107 qualitativ auf das Vorhandensein von Schimmel. Die Steuereinheit 107 erfasst somit also qualitativ das Vorhandensein von Schimmel in allen drei untersuchten Bereichen 102a, 102b, 102c der Baustoffoberfläche 101.

Basierend auf dieser qualitativen Erfassung von Schimmel in den zu untersuchenden Bereichen 102a, 102b, 102c bestimmt die Steuereinheit 107 anschließend quantitativ die Schimmelmenge in zumindest einem Teil der Baustoffoberfläche 101. Hierfür kann die Steuereinheit 107 beispielsweise quantitativ die Schimmelmenge in mindestens einem, vorzugsweise aber in allen drei zu untersuchenden Bereichen 102a, 102b, 102c bestimmen. Da jeder der drei zu untersuchenden Bereiche 102a, 102b, 102c in dem in Figur 2 gezeigten Beispiel kleiner ist als die gesamte Baustoffoberfläche 101, bestimmt die Steuereinheit 107 somit also quantitativ die Schimmelmenge auf zumindest einem Teil der Baustoffoberfläche 101, nämlich auf demjenigen Teil der Baustoffoberfläche 101, der der Größe der untersuchten Bereiche 102a, 102b, 102c entspricht. Die Bestimmung der Schimmelmenge kann in allen zu untersuchenden Bereichen 102a, 102b, 102c gleichzeitig, oder sequentiell, d.h. nacheinander erfolgen.

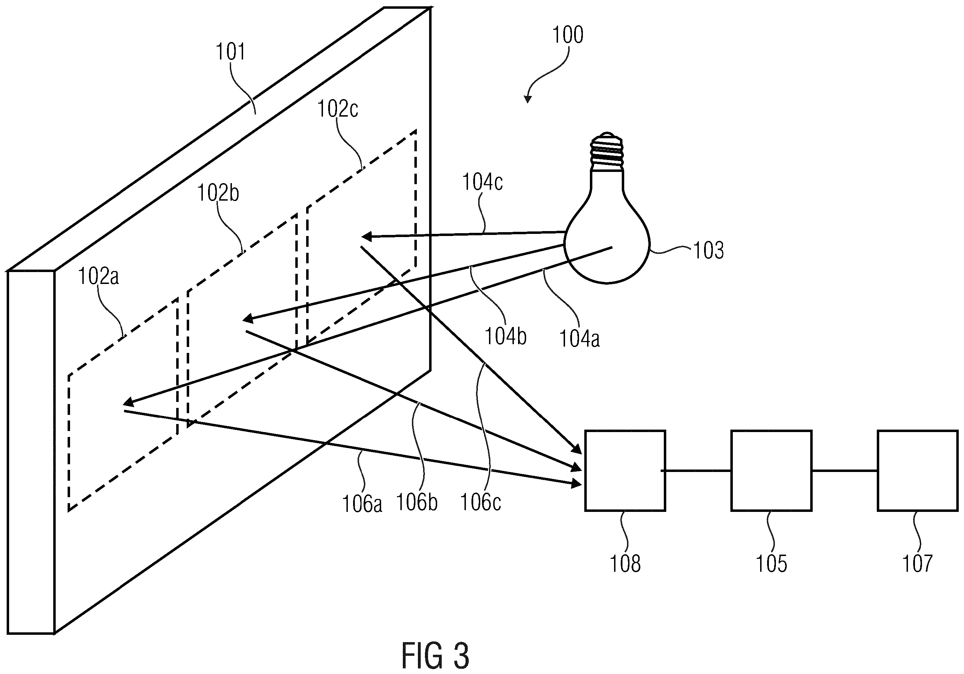

Figur 3 zeigt ein weiteres Beispiel einer Vorrichtung 100 zum Erfassen von Schimmel. Diese Vorrichtung entspricht im Wesentlichen der zuvor mit Bezug auf Figur 2 diskutierten Beispiel, mit dem Unterschied, dass vor dem Lichtempfänger 105 eine spektral auflösende Optik 108 angeordnet ist.

Die spektral auflösende Optik 108 ist ausgebildet, um das empfangene Licht 106 in sein Spektrum zu zerlegen. Die Steuereinheit 107 ist ausgebildet, um dieses Spektrum dahingehend zu überprüfen, ob das empfangene Licht 106 zumindest anteilig von Schimmel fluoresziertes Licht aufweist.

Falls Schimmel in einem zu untersuchenden Bereich 102 vorhanden sein sollte, wird dieser bei Bestrahlung mit UV-Licht 104 fluoreszieren. Das von dem Schimmel fluoreszierte Licht 104, sowie möglicher Weise weitere Lichtbestandteile anderer Wellenlängen, werden von der spektral auflösenden Optik 108 empfangen. Die spektral auflösende Optik 108 zerlegt dann das empfangene Licht 106 in sein Spektrum. Ein solches Spektrum kann beispielsweise in einem Spektrogramm abgebildet werden.

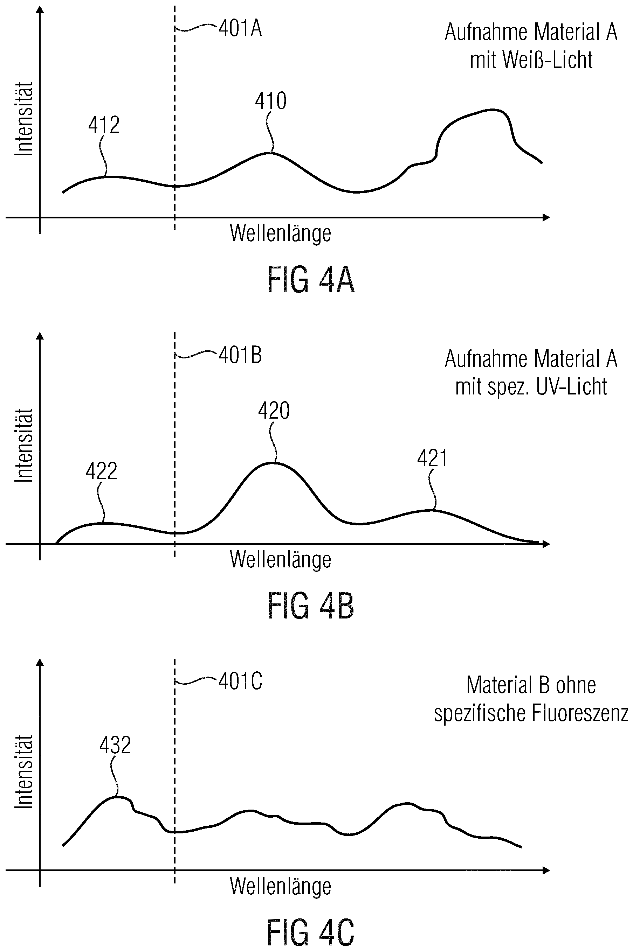

Die Figuren 4A bis 4C zeigen beispielhafte Spektrogramme 400A, 400B, 400C, bei denen die Intensität über der Wellenlänge des zerlegten Lichts aufgetragen ist. In die Spektrogramme 400A, 400B, 400C ist jeweils eine in Strichlinien exemplarisch dargestellte Grenzlinie 401A, 401B, 401C eingezeichnet. Links von dieser Grenzlinie 401A, 401B, 401C befindet sich exemplarisch der UV-Bereich.

Der UV-Bereich ist hier lediglich qualitativ eingezeichnet. Quantitativ erstreckt sich der in der Erfindung nutzbare UV-Bereich von 380 nm bis hinunter zu einer Wellenlänge von 10 nm. Vorzugsweise werden Wellenlängen im nahen UV-Bereich mit einer Wellenlänge von 380 nm bis 315 nm genutzt. Weitere vorzugsweise nutzbaren Wellenlängenbereiche erstrecken sich von 315 nm bis 281 nm, sowie von 280 nm bis 200 nm.

Die in den Figuren 4A, 4B, 4C abgebildeten Spektrogramme sind beispielsweise an drei verschiedenen Baustoffoberflächen aufgenommen worden. Figur 4A zeigt ein Reflexions-Spektrum eines mit breitbandigem Weißlicht beleuchteten, aus dem Material A bestehenden, Prüfobjekts. Im Bereich 410 ist ein Fluoreszenz-Effekt sichtbar. Figur 4B zeigt das Reflexions-Spektrum eines aus dem gleichen Material A bestehenden Prüfobjekts, jedoch nur mit reinem UV-Licht beleuchtet. Die Intensität der Reflexion wird im Bereich 422, im Vergleich zu dem Bereich 412, absehbar steigen. Der Anstieg ist abhängig von der Intensität der Lichtquellen. Im Bereich 420 steigt die Reflexion, im Vergleich zu dem Bereich 410, deutlich wegen des im sichtbaren Wellenlängenbereich sichtbaren Fluoreszenz-Effektes an. Wegen der geringen, oder möglicherweise fehlenden, Beleuchtung im sichtbaren Wellenlängenbereich wird die Reflexion 421 hier im Vergleich zu Figur 4A entsprechend geringer ausfallen. Figur 4C zeigt ein Spektrum mit materialspezifischer Reflexion im UV-Bereich 432, jedoch ohne, oder gegebenenfalls mit nur sehr geringem, Fluoreszenz-Effekt.

Die Steuereinheit 107 wertet die jeweiligen Spektren aus. Die Steuereinheit 107 ist ausgebildet, um anhand der im UV-Wellenlängenbereich detektierten Signale auf das Vorhandensein von Schimmel zu schließen. Beispielsweise zeigt Figur 4A ein für eine erste Schimmelart charakteristisches Spektrum mit Signalanteilen im UV-Bereich. Figur 4B hingegen zeigt ein für eine zweite Schimmelart charakteristisches Spektrum mit Signalanteilen im UV-Bereich. Figur 4C zeigt ebenfalls ein Spektrum mit Signalanteilen im UV-Wellenlängenbereich, die jedoch für keine Schimmelart charakteristisch sind. Dieses in Figur 4C gemessene Spektrum im UV-Bereich könnte beispielsweise auf eine andere Fluoreszenzquelle schließen lassen, die jedoch kein Schimmel ist.

Die Steuereinheit 107 kann also das von der spektralen Optik 108 erzeugte Spektrum dahingehend überprüfen, ob das empfangene Licht zumindest anteilig von Schimmel fluoresziertes Licht aufweist. Für einige Schimmelarten sind charakteristische Fluoreszenz-Effekte bekannt. Daher kann man starke Reflexionen in, für die jeweilige Schimmelart, spezifischen Bereichen als Indikator für Schimmel nutzen. Der Vergleich der Reflexionen durch Weiß-Licht, insbesondere durch breitbandiges Weiß-Licht, und speziellem UV-Licht verstärkt die Sichtbarkeit dieses Effekts, wie zuvor mit Bezug auf die Figuren 4A und 4B näher beschrieben wurde. Die Steuereinheit 107 bestimmt somit qualitativ das Vorhandensein von Schimmel in dem jeweils untersuchten Bereich 102. Die Schimmelmenge kann quantitativ beispielsweise anhand der Intensitätsverteilung in einem Spektrogramm ermittelt werden.

Die Steuereinheit 107 kann außerdem dazu ausgebildet sein, um eine Absorption und/oder Reflexion bestimmter Wellenlängenbereiche in dem empfangenen Licht 106 des zu untersuchenden Bereichs 102 zu erfassen und diese absorbierten und/oder reflektierten Wellenlängenbereiche als Identifikationsmerkmal zur Bestimmung der Schimmelart heranzuziehen. Mit Bezug auf die Figuren 4A und 4B ist zu erkennen, dass die jeweils abgebildeten Spektren 400A, 400B auch außerhalb des UV-Bereichs, d.h. rechts der Grenzlinie 401A, 401B, eine Absorption von Licht unterschiedlicher Wellenlängenbereiche zeigen. So weist das Spektrum 400A eine Verteilung auf, die charakteristisch für eine Schimmelart A ist. Das Spektrum 400B hingegen weist eine Verteilung auf, die charakteristisch für eine Schimmelart B ist. So kann die Steuereinheit 107 anhand der Spektralverteilung die Schimmelart bestimmen.

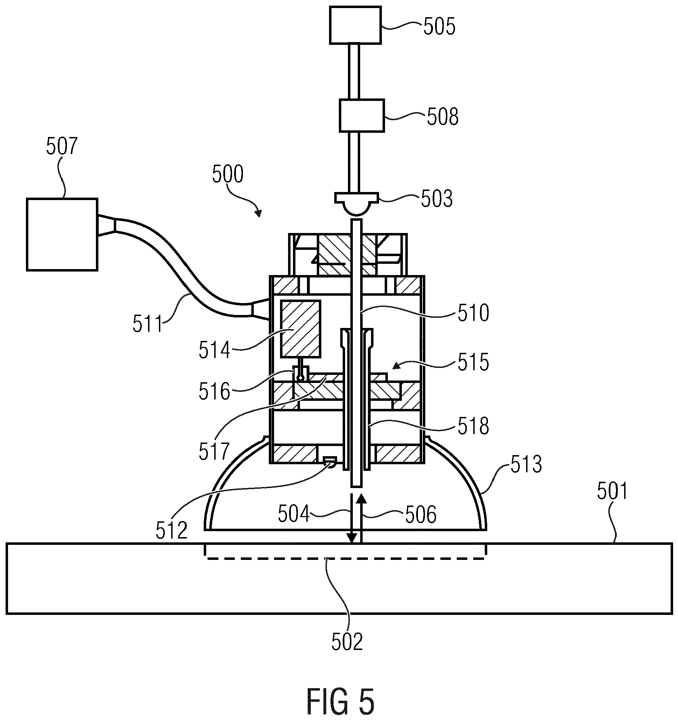

Figur 5 zeigt eine Ausführungsform einer erfindungsgemäßen Vorrichtung 500. Die Vorrichtung 500 weist eine beispielsweise als LED oder als Halogenlichtquelle ausgebildete Primärlichtquelle 503 auf. Die Lichtquelle 503 ist ausgebildet, um zumindest teilweise UV-Licht 504 auszusenden.

Die Vorrichtung 500 weist einen Lichtwellenleiter 510 auf. Der Lichtwellenleiter 510 ist mit der Lichtquelle 503 optisch gekoppelt. Das von der Lichtquelle 503 ausgesendete Licht 504 wird in den Lichtwellenleiter 510 eingekoppelt und auf zumindest einen Abschnitt eines zu untersuchenden Bereichs 502 der Baustoffoberfläche 501 geleitet.

Die Vorrichtung 500 weist außerdem einen Lichtempfänger 505 auf. Der Lichtempfänger 505 kann ebenfalls optisch mit dem Lichtwellenleiter 510 gekoppelt sein. Der Lichtempfänger 505 kann aber auch mit einem anderen, in Figur 5 nicht dargestellten Lichtwellenleiter, optisch gekoppelt sein, der das von der Baustoffoberfläche 101 ausgehende Licht 506 zu dem Lichtempfänger 505 leitet.

Das von dem zu untersuchenden Bereich 502 ausgehende Licht 506 wird in den Lichtwellenleiter 510 eingekoppelt. Der Lichtwellenleiter 510 leitet das eingekoppelte Licht 506 zu dem Lichtempfänger 505 weiter.

Die Vorrichtung 500 weist außerdem eine spektral auflösende Optik 508 auf. Die spektral auflösende Optik 508 ist vor dem Lichtempfänger 505 angeordnet. Die spektral auflösende Optik 508 kann auch an einem dem zu untersuchenden Bereich 502 gegenüberliegend angeordneten Ende des Lichtwellenleiters 510 angeordnet sein. Die spektral auflösende Optik 508 und der Lichtempfänger 505 können auch als eine gemeinsame Einheit ausgebildet sein.

Der Lichtempfänger 505 kann beispielsweise als ein Punktsensor 505 ausgebildet sein. Der Punktsensor 505 bzw. die Größe oder Fläche des Punktsensors 505 bestimmt einen Bildpunkt bzw. die Größe dieses Bildpunkts innerhalb des zu untersuchenden Bereichs 502. Die spektral auflösende Optik 508 ist ausgebildet, um das in diesem einen Bildpunkt empfangbare Licht in sein Spektrum zu zerlegen. In anderen Worten wird der zu untersuchende Bereich 502 pixelweise auf Vorhandensein von Schimmel untersucht.

In einer alternativen Ausgestaltung kann der Lichtempfänger 505 als ein Flächensensor 505 ausgebildet sein. Der Flächensensor 505 weist vorzugsweise eine Größe zwischen 2x2 und mehr Bildpunkten auf. Die Größe der Fläche des Flächensensors 505 bestimmt die Größe des Bildbereichs innerhalb des zu untersuchenden Bereichs 502. Die spektral auflösende Optik 508 ist ausgebildet, um das in diesem Bildbereich empfangbare Licht in sein Spektrum zu zerlegen. In anderen Worten wird der zu untersuchende Bereich 502 bildflächenweise, entsprechend der Größe der Sensorfläche, auf Vorhandensein von Schimmel untersucht.

In einer alternativen, hier nicht dargestellten Ausführungsform, kann der Lichtwellenleiter 510 entfallen. Der Lichtempfänger 505 wäre in diesem Fall über dem zu untersuchenden Bereich 502 angeordnet, sodass der Lichtempfänger 505 direkten Sichtkontakt zu dem zu untersuchenden Bereich 502 hat. Die Reflexionen des zu untersuchenden Bereichs 502 gelangen dann unmittelbar, d.h. unter Umgehung der Einkopplung des Lichts in den Lichtwellenleiter 510, auf den Lichtempfänger 505. Der Lichtempfänger 505 könnte beispielsweise an einem dem zu untersuchenden Bereich 102 zugewandten Abschnitt 519 der Vorrichtung 500 angebracht sein.

Die Vorrichtung 500 weist ferner eine Steuereinheit 507 auf. Die Steuereinheit 507 ist mit der Vorrichtung 500 über eine Signalleitung 511 verbunden. Die Steuereinheit 507 kann vor Ort an der Vorrichtung 500 vorgesehen sein. Es ist aber auch denkbar, dass die Steuereinrichtung 507 über eine Signalleitung 511, wie beispielsweise einer Netzwerkleitung, von der Vorrichtung 500 entfernt vorgesehen ist. Beispielsweise kann die Steuereinrichtung 507, bzw. die Auswertefunktionalität der Steuereinrichtung 507, auf einem entfernten Rechner, einem Server bzw. in einer Cloud integriert sein. Die Signalleitung 511 kann eine drahtgebundene oder eine drahtlose Signalverbindung sein. Die Signalverbindung kann per Hardware, z.B. mittels einem Kabel, oder kabellos, z.B. mittels W-LAN, Bluetooth, oder anderen Funkstandards, erfolgen. Weiter können Steuereinheit 507 und Lichtempfänger 505 auch in einer gemeinsamen Einheit, z.B. auf einer Platine, aufgebaut sein. Die Steuereinheit 507 kann real, z.B. als ein eigener Rechner, oder virtuell, z.B. in einer Cloud, sein.

Eine beispielhafte Steuereinheit kann ausgebildet sein, um das mit dem Lichtempfänger 505 empfangene Licht 506 dahingehend zu überprüfen, ob das empfangene Licht 506 zumindest anteilig fluoresziertes Licht aufweist, was wiederum, wie zuvor mit Bezug auf die Figuren 1 bis 4 beschrieben, auf das Vorhandensein von Schimmel hindeuten kann. Prinzipiell weist die Steuereinheit 507 dieselbe Funktionalität auf wie die mit Bezug auf die Figuren 1 bis 4 beschriebene Steuereinheit 107.

Die Vorrichtung 500 weist eine zweite Lichtquelle 512 auf. Die zweite Lichtquelle 512 ist vorzugsweise als LED ausgebildet. Die LED 512 kann Licht im sichtbaren und/oder im UV-Bereich aussenden. Die LED 512 kann auch als primäre bzw. einzige Lichtquelle 503 genutzt werden, vorausgesetzt sie ist ausgebildet, um zumindest UV-Licht auszusenden.

In dem in Figur 5 abgebildeten Ausführungsbeispiel ist die Lichtquelle 503 eine Halogenlichtquelle, und die zweite Lichtquelle 512 ist als eine UV-Licht emittierende LED ausgebildet. Beide Lichtquellen 503, 512 sind sequentiell schaltbar, d.h. sie sind nacheinander ein- und ausschaltbar. So kann der vorzugsweise als Punktsensor oder Flächensensor ausgebildete Lichtempfänger 505 Aufnahmen des zu untersuchenden Bereichs 502 in unterschiedlichen Beleuchtungsszenarien machen. Genauer gesagt macht der Lichtempfänger 505 eine erste Aufnahme von dem zu untersuchenden Bereich 502 wenn dieser mit der ersten Lichtquelle 503, d.h. mit Halogenlicht, beleuchtet ist. Nach dem Umschalten von der ersten Lichtquelle 503 auf die zweite Lichtquelle 512 macht der Lichtempfänger 505 eine zweite Aufnahme von dem zu untersuchenden Bereich 502, wenn dieser mit der zweiten Lichtquelle 503, d.h. mit UV-Licht, beleuchtet ist.

Die anspruchsgemäße Steuereinheit 507 ist ausgebildet, um die erste Aufnahme mit der zweiten Aufnahme zu vergleichen. Ein Vergleich der ersten Aufnahme mit der zweiten Aufnahme kann zur besseren Erfassung von Schimmel dienen. Beispielsweise können auf dem zu untersuchenden Bereich 502 vorhandene Fremdanteile, die nicht von Schimmel herrühren, im sichtbaren Halogenlicht besser erkannt werden, als im UV-Licht. Nicht-fluoreszierende Anteile von Schimmel können beispielsweise ebenfalls besser unter sichtbarem Licht als unter UV-Licht erkannt werden.

Generell emittieren die beiden Lichtquellen 503, 512 Licht unterschiedlicher Wellenlänge und somit unterschiedlicher Farbe, sofern zumindest eine der beiden Lichtquellen 503, 512 UV-Licht aussendet. Es kann aber auch ausreichend sein, wenn lediglich eine schaltbare Lichtquelle 503 vorgesehen ist, die sowohl UV-Licht, als auch Licht in weiteren Wellenlängenbereichen aussenden kann, sofern der Lichtempfänger 505 zumindest eine Aufnahme mit Beleuchtung des zu untersuchenden Bereichs unter UV-Licht macht. So kann die Steuereinheit 507 diese mit UV-Licht beleuchtete Aufnahme mit einer weiteren, unter einer anderen Beleuchtungssituation, gemachten Aufnahme vergleichen.

Die Vorrichtung 500 weist ferner eine Kuppel 513 auf. Die Kuppel 513 ist vorzugsweise lichtundurchlässig ausgebildet, d.h. sie dient als Lichtschutz und sorgt dafür, dass kein bzw. lediglich ein nicht messwertverfälschender Lichtanteil von außen zu dem zu untersuchenden Bereich 502 dringt. Die Kuppel 513 umschließt das der Baustoffoberfläche 501 zugewandte Ende der Vorrichtung 500 lichtdicht. Das der Baustoffoberfläche 501 zugewandte Ende der Kuppel 513 kann lichtdicht auf der Baustoffoberfläche 501 aufgesetzt werden.

Die Vorrichtung 500 weist eine Positionierungseinheit 515 zum Positionieren des Lichtwellenleiters 510 mit eingekoppelter Primärbeleuchtung auf. Die Positionierungseinheit 515 kann die Vorrichtung 500 bzw. den Lichtwellenleiter 510 auf einer Bahn bewegen. Die Positionierungseinheit 515 weist einen Antrieb 514, ein Getriebe 516 und eine Positionierung bzw. eine verschiebbare Platte 517 auf. Die verschiebbare Platte 517 ist zweidimensional bewegbar bzw. verfahrbar. Die zweidimensionale Bewegung entspricht einer Bewegung in die Bildebene von Figur 5 hinein bzw. aus dieser heraus, sowie links und rechts entlang der in Figur 5 gezeigten Baustoffoberfläche 501. In anderen Worten kann die verschiebbare Platte 517 parallel zu der Baustoffoberfläche 501 bewegbar sein.

In der verschiebbaren Platte 517 ist eine Aufnahmevorrichtung 518 integriert. Die Aufnahmevorrichtung 518 dient zum Aufnehmen des Lichtwellenleiters 510. Sobald der hier als Elektromotor ausgebildete Antrieb 514 das Getriebe 516 betätigt, verfährt die verschiebbare Platte 517 zweidimensional in eine Richtung. Die mit der verschiebbaren Platte 517 integrierte Aufnahmevorrichtung 518 bewegt sich ebenfalls in diese Richtung und überträgt diese Bewegung auf den darin aufgenommenen Lichtwellenleiter 510. Der Lichtwellenleiter 510 kann so zum Aufnehmen des von dem zu untersuchenden Bereich 502 ausgehenden Lichts 506 an verschiedene Positionen innerhalb des zu untersuchenden Bereichs 502 verfahren werden.

In einem alternativen, hier nicht dargestellten, Beispiel kann der Lichtwellenleiter 510 entfallen. In diesem Fall kann der Lichtempfänger 505 dem zu untersuchenden Bereich 502 gegenüberliegend angeordnet sein, sodass der Lichtempfänger 505 direkten Sichtkontakt zu dem zu untersuchenden Bereich 502 hat. Der Lichtempfänger 505 kann, entweder unmittelbar oder mittelbar, z.B. mittels der Aufnahmevorrichtung 518, mit der Positioniervorrichtung 515 verbunden sein.

Somit kann der vorzugsweise als Punktsensor oder Flächensensor ausgebildete Lichtempfänger 505 bewegbar an der Vorrichtung 500 angeordnet sein. Genauer gesagt kann der Lichtempfänger 505 mittels der Positioniervorrichtung 515, wie zuvor beschrieben, zweidimensional bewegbar bzw. verfahrbar sein. Der Lichtempfänger 505 kann so mit direktem Sichtkontakt auf die Baustoffoberfläche 501 parallel zu der Baustoffoberfläche 501 bewegbar sein. Der Lichtempfänger 505 kann somit die Baustoffoberfläche 501 bildlich abtasten bzw. scannen.

Der Lichtempfänger 505 kann parallel zu einem zu untersuchenden Bereich 502 verfahrbar sein. Der Lichtempfänger 505 kann vorzugsweise innerhalb eines gesamten zu untersuchenden Bereichs 502 verfahrbar sein, um den gesamten zu untersuchenden Bereich 502 bildlich abtasten zu können.

Die Vorrichtung 500 nimmt Stichproben von dem zu untersuchenden Bereich 502. Das heißt, der Lichtempfänger 505 wird an diejenigen ausgewählten vordefinierten Positionen, an denen die Stichproben genommen werden sollen, innerhalb des zu untersuchenden Bereichs 502 verfahren. Wenn die Steuereinheit 507 beispielsweise fünf Positionen innerhalb des zu untersuchenden Bereichs 502 bestimmt hat, an denen Stichproben genommen werden sollen, so wird der Lichtempfänger 505 nacheinander an diese ausgewählten fünf Positionen verfahren. An jeder Position kann der Lichtempfänger 505 eine oder mehrere Aufnahmen des jeweiligen Teils der Baustoffoberfläche 501 erstellen.

Vorzugsweise werden an jeder Position des Lichtempfängers 505 Aufnahmen mit unterschiedlicher Beleuchtung gemacht. So werden beispielsweise an jeder Position eine Aufnahme mit UV-Licht und eine oder mehrere weitere Aufnahmen mit breitbandigerem Licht und/oder mit Licht anderer Wellenlänge gemacht.

Dies bietet den Vorteil, dass die Vorrichtung 500 lediglich initial auf die Baustoffoberfläche 501 bzw. auf den zu untersuchenden Bereich 502, aufgesetzt wird, und die Untersuchung der Baustoffoberfläche 502 weitestgehend automatisiert erfolgt. Die Kuppel 513 umgibt den zu untersuchenden Bereich 502 vorzugsweise lichtdicht. Der Steuereinheit 507 berechnet anhand bekannter, z.B. zuvor eingegebener Daten, die Anzahl und Positionen der zu nehmenden Stichproben. Die Steuereinheit 507 steuert die Positioniervorrichtung 515 entsprechend an. Die Positioniervorrichtung 515 fährt den Lichtempfänger 505 an die entsprechende Position.

Die Lichtquelle 503 bzw. die mehreren Lichtquellen 503, 512, beleuchten den zu untersuchenden Bereich 502 mit entsprechendem Licht. Der Lichtempfänger 505 macht eine Aufnahme des für den Lichtempfänger 505 sichtbaren Teils des zu untersuchenden Bereichs 502 bei entsprechender Beleuchtung. Anschließend wird der Lichtempfänger 505 zu der nächsten Position verfahren, um dort ebenfalls weitere Aufnahmen zu machen.

Die Messungen bzw. die mittels des Lichtempfängers 505 getätigten Aufnahmen sind reproduzierbar. Das heißt, Stichproben können bei Bedarf mehrmals unter Einhaltung der gleichen Randbedingungen, d.h. unter Einhaltung der ermittelten Positionen sowie der Anzahl und den Beleuchtungsszenarien, genommen werden.

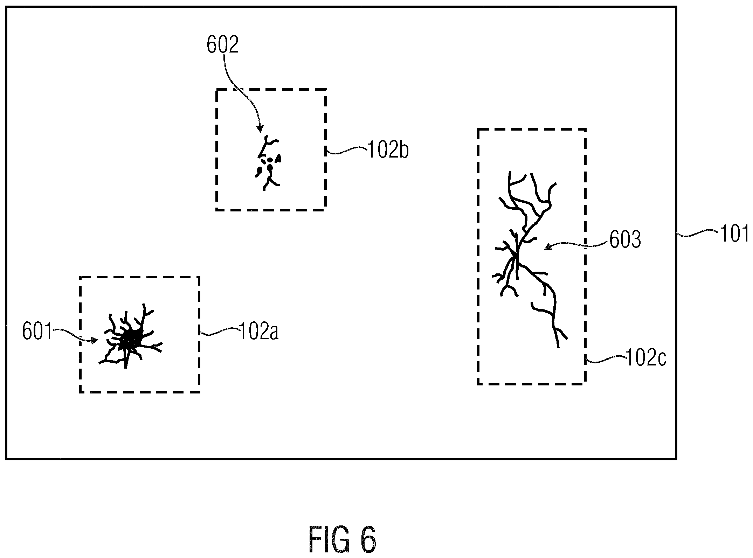

Figur 6 zeigt einen Ausschnitt einer Baustoffoberfläche 101. Die Baustoffoberfläche 101 ist an drei unterschiedlichen Stellen 601, 602, 603 mit Schimmel kontaminiert. Diese drei unterschiedlichen Stellen 601, 602, 603 sind bekannte Hauptansiedlungspunkte von Schimmel, beispielsweise weil diese Stellen besonders hoher Feuchtigkeit oder anderen Schimmelwachstum begünstigenden Faktoren unterliegen.

Die Steuereinheit 107, 507 (Figuren 1, 2, 3 und 5) ist ausgebildet, um für eine stichprobenartige Bestimmung der Schimmelmenge eines Teils der Baustoffoberfläche 101, die Baustoffoberfläche 101 in eine Anzahl zu untersuchender Bereiche 102a, 102b, 102c zu unterteilen, wobei die Anzahl zu untersuchender Bereiche 102 sowie eine Anzahl in den jeweiligen zu untersuchenden Bereichen 102 durchzuführender Stichproben basierend auf bekannten Hauptansiedlungspunkten 601, 602, 603 des Schimmels und bekannten Ausbreitungscharakteristiken des Schimmels bestimmt wird.

In anderen Worten sind der Steuereinheit 107, 507 die auf der Baustoffoberfläche 101 vorhandenen Hauptansiedlungspunkte 601, 602, 603 bekannt. Der Steuereinheit 107, 507 sind außerdem die Ausbreitungscharakteristiken verschiedener Schimmelarten bekannt. Beispielsweise kann die Steuereinheit 107, 507 eine Eingabevorrichtung aufweisen. Mittels dieser Eingabevorrichtung können von einem Nutzer entsprechende Informationen, wie z.B. eine Klassifizierung, eine bestimmte Menge und ein bestimmter Ort des Schimmels eingegeben werden.

Wie in Figur 6 zu sehen ist, weist die in dem ersten bekannten Hauptansiedlungspunkt 601 auftretende Schimmelart ein stark bewachsenes Zentrum auf, wobei sich die von diesem Zentrum ausgehenden Hyphen des Schimmelpilzes in alle Richtungen etwa gleichmäßig bzw. etwa konzentrisch ausbreiten.

Die in dem zweiten Hauptansiedlungspunkt 602 auftretende Schimmelart weist im Zentrum mehrere kleine, sporenartige Ansammlungen auf. Die Hyphen verteilen sich von diesen einzelnen Zentren aus nach außen.

Die in dem dritten Hauptansiedlungspunkt 603 auftretende Schimmelart weist ein fadenförmiges Zentrum auf. Ausgehend von diesem fadenförmigen Zentrum verteilen sich die Hyphen beispielsweise in eine bevorzugte Ausbreitungsrichtung, beispielsweise zwischen der Oberund Unterkante der Baustoffoberfläche 101.

Basierend auf diesen Hauptansiedlungspunkten 601, 602, 603 und den bekannten Ausbreitungscharakteristiken des Schimmels kann die Steuereinheit 107, 507 die Baustoffoberfläche 101 in eine bestimmte Anzahl von zu untersuchenden Bereichen 102a, 102b, 102c unterteilen. Ebenso kann die Steuereinheit 107, 507 die Form und Größe eines zu untersuchenden Bereichs 102a, 102b, 102c bestimmen.

So weisen beispielsweise der in dem ersten Hauptansiedlungspunkt 601 bestimmte zu untersuchende Bereich 102a sowie der in dem zweiten Hauptansiedlungspunkt 602 bestimmte zu untersuchende Bereich 102b eine etwa quadratische Form auf, da sich die in diesen Bereichen befindlichen Schimmelpilze etwa gleichmäßig in alle Richtungen ausbreiten.

Der in dem dritten Hauptansiedlungspunkt 603 bestimmte zu untersuchende Bereich 102c weist eine etwa langgestreckte rechteckige Form auf, da sich der in diesem Bereich befindliche Schimmelpilz etwa fadenförmig in eine bevorzugte Ausbreitungsrichtung erstreckt.

Die Steuereinheit 107, 507 ist ausgebildet, um die Schimmelmenge in den jeweiligen zu untersuchenden Bereichen 102a, 102b, 102c stichprobenartig zu bestimmen. Ebenfalls basierend auf den bekannten Hauptansiedlungspunkten sowie den bekannten Ausbreitungscharakteristiken des jeweiligen Schimmelpilzes bestimmt die Steuereinheit 107, 507 die Anzahl der durchzuführenden Stichproben. Die Steuereinheit 107, 507 bestimmt außerdem die Positionen innerhalb eines zu untersuchenden Bereichs 102a, 102b, 102c, an denen Stichproben genommen werden.

Die innerhalb eines zu untersuchenden Bereichs 102a, 102b, 102c genommenen Stichproben werden dann von der Steuereinheit 107, 507 statistisch ausgewertet. Die Steuereinheit 107, 507 bestimmt, basierend auf einer Extrapolation der Stichproben, die statistische Verteilungswahrscheinlichkeit des Schimmelpilzes innerhalb eines gesamten zu untersuchenden Bereichs 102a, 102b, 102c. Die Steuereinheit 107, 507 bestimmt somit quantitativ die Schimmelmenge auf einem Teil der Baustoffoberfläche 101. In diesem Fall bestimmt die Steuereinheit 107, 507 quantitativ die Schimmelmenge auf denjenigen Teilen der Baustoffoberfläche 101, die der Größe der zu untersuchenden Bereiche 102a, 102b, 102c entsprechen.

Die Steuereinheit 107, 507 ist außerdem ausgebildet, um die Ergebnisse aller zu untersuchenden Bereiche 102a, 102b, 102c zu extrapolieren und so eine statistische Verteilungswahrscheinlichkeit von Schimmel auf der gesamten Baustoffoberfläche 101 zu berechnen. Die Steuereinheit 107, 507 bestimmt somit quantitativ die Schimmelmenge auf der gesamten Baustoffoberfläche 101. Diese Hochrechnung auf die gesamte Baustoffoberfläche 101 ist umso genauer, je größer der Anteil an der von den zu untersuchenden Bereichen 102a, 102b, 102c eingenommenen Fläche gegenüber der Gesamtfläche der Baustoffoberfläche 101 ist.

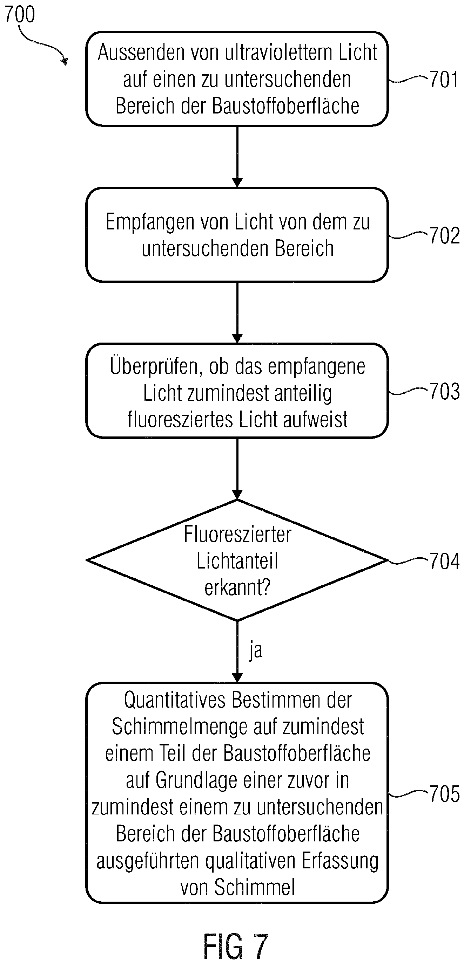

Figur 7 zeigt ein Ablaufdiagramm eines beispielhaften Verfahrens 700 zum Erfassen und Bestimmen einer Menge von unter Bestrahlung mit ultraviolettem Licht fluoreszierendem Schimmel auf Baustoffoberflächen. Das erfindungsgemäße Verfahren wird jedoch durch Anspruch 10 definiert.

In Schritt 701 wird erfindungsgemäß ultraviolettes Licht 104 auf einen zu untersuchenden Bereich 102 der Baustoffoberfläche 101 ausgesendet. Dabei kann es sich um reines UV-Licht, oder um breitbandigeres Licht mit einem UV-Anteil handeln.

In Schritt 702 wird erfindungsgemäß Licht 106, das von dem zu untersuchenden Bereich 102 ausgeht, empfangen. Bei diesem von dem zu untersuchenden Bereich 102 ausgehenden Licht kann es sich, zumindest teilweise, um von dem zu untersuchenden Bereich 102 reflektiertes Licht und/oder um von Objekten, wie insbesondere Schimmel, ausgesendetes fluoresziertes Licht handeln.

In Schritt 703 wird erfindungsgemäß überprüft, ob das empfangene Licht 106 zumindest anteilig fluoresziertes Licht aufweist. Fluoresziertes Licht ist Licht im sichtbaren Bereich. Eine solche Überprüfung kann zum Beispiel durch einen Vergleich eines Reflexions-Spektrums des empfangenen Lichts 106 mit bekannten, für Schimmel charakteristischen Reflexions-Spektren erfolgen. Hierbei kann beispielsweise mittels der Steuereinrichtung verglichen werden, ob das Reflexions-Spektrum des empfangenen Lichts 106 mit einem bekannten, für Schimmel charakteristischen Reflexions-Spektrum zu einem gewissen Grad übereinstimmt. Eine Übereinstimmung kann auf das Vorhandensein von Schimmel hindeuten. Wenn außerdem bekannt ist, dass in diesem bekannten, für Schimmel charakteristischen Reflexions-Spektrum Fluoreszenzanteile vorhanden sind, so kann die Steuereinrichtung ferner darauf schließen, dass auch in dem Reflexions-Spektrum des empfangenen Lichts 106 Fluoreszenzanteile vorhanden sein müssen.

In Block 704 wird eine Entscheidung getroffen. Falls ein fluoreszierter Lichtanteil erkannt wurde, kann auf das Vorhandensein von Schimmel geschlossen werden. Das empfangene Lichtspektrum, d.h. die Verteilung der Intensität über die Wellenlänge, kann beispielsweise einem für eine bestimmte Schimmelart charakteristischen Spektrum entsprechen. Falls beispielsweise ein Spektrum einer Schimmelart mit fluoreszierenden Eigenschaften erkannt wird, kann darauf geschlossen werden, dass das empfangene Licht zumindest Anteile von Fluoreszenz aufweist. Somit kann beispielsweise ein fluoreszierter Lichtanteil erkannt werden. Es erfolgt hier also eine qualitative Erfassung von Schimmel. Falls in Block 704 eine qualitative Erfassung von Schimmel anhand des erkannten Fluoreszenzlichtanteils stattgefunden hat, wird zu Schritt 705 übergegangen.

In Schritt 705 wird erfindungsgemäß die Schimmelmenge auf zumindest einem Teil der Baustoffoberfläche 101 quantitativ bestimmt, und zwar auf Grundlage der zuvor (Block 704) in zumindest einem zu untersuchenden Bereich 102 der Baustoffoberfläche 101 ausgeführten qualitativen Erfassung von Schimmel.

Zusammenfassend beinhaltet die Erfindung die folgenden Merkmale:
Die Erfindung nutzt die sich verändernde spektrale Reflexion der Oberfläche 101 durchunterschiedliche Schimmelkontaminationen. Die spezifische Reflexion kann durch fluoreszierende Effekte (Beleuchtung/Anregung im UV, sichtbar im sichtbaren Wellenlängenbereich (VIS)) verstärkt werden. Diese fluoreszierenden Effekte werden bis heute nicht für in situ Messungen in Echtzeit verwendet.

Erfindungsgemäß wird daher die spektrale Reflexion durch einen optischen Sensor erfasst und dann mit entsprechenden Algorithmen ausgewertet.

Die Erfindung beinhaltet die Messung der Schimmelkontamination auf Bauwerks-Oberflächen 101 und dem oberflächennahen Porenraum von Baustoffen. Die Erfindung weist die in den unabhängigen Ansprüchen definierten Merkmale/Verfahrensschritte auf und kann durch die folgenden beispielhaften Bestandteile implementiert werden:
- Messeinheit / Sensor zur Detektion der Schimmelkontamination => Messeinheit zum Nachweis und zur Quantifizierung von Schimmelsporen und Schimmelwachstum (Schimmelmyzel) auf Bauwerks-Oberflächen 101; Die Messung erfolgt in Echtzeit (rechtzeitig) und in situ mit optischen Verfahren. Die verwendete Beleuchtung nutzt Fluoreszenz-Effekte bei Schimmel (UV-Beleuchtung) und ist vorzugsweise schaltbar; die Detektion erfolgt vorzugsweise mittels (spektral auflösender) Optik 108 (in der Regel im VIS), z.B. eines Spektrometers. Zudem kann auch die Absorption bestimmter Wellenlängenbereiche (UV bis SWIR) als zusätzliches Identifikationsmerkmal herangezogen werden. Hierfür wird eine spektral breitbandige Beleuchtung 103 eingesetzt, die den Messbereich des Spektroskops 108 überdeckt. Ebenso kann gegebenenfalls der Aufnahmebereich (aufgenommene Wellenlängen) durch geeignete Filter eingeschränkt werden, um so bestimmte Effekte (z. B. Fluoreszenz) für die Detektion zu verstärken.
- Algorithmen zur Verarbeitung der mit dem Sensor aufgenommenen Messdaten => Durch die Möglichkeit, den Untersuchungsbereich 102, 502 mit einer oder mehreren verschiedenen Lichtquellen 103, 503, 512 (in der Regel homogene Lichtquellen) zu beleuchten, können zur Verbesserung der Ergebnisse die Aufnahmen miteinander verglichen und mittels Algorithmen ausgewertet werden. Die zu vergleichenden Aufnahmen sind vorzugsweise homogen und in Wellenlängen des sichtbaren (VIS) und/oder UV-Licht beleuchtet.
- Auswertesystematik zur Ermittlung der Kontaminationsmenge einer Oberfläche 101 mit statistischen Methoden => zur Bestimmung der Kontamination einer Fläche 101 wird ein statistisches Modell der Verteilung verwendet. Hierfür wird die Fläche 101 in eine Anzahl festzulegender Quadranten 102a, 102b, 102c aufgeteilt. Auf der Basis bekannter "Haupt-Kontaminationspunkte" der Fläche 101 und der bekannten Entwicklungsmechanismen der Schimmelkontamination werden dann Anzahl und Positionen (welche Quadranten 102a, 102b, 102c und Anzahl der Messepunkte im Quadranten) für die notwendigen Stichproben festgelegt. Als Ergebnis wird dann die erwartete Kontamination der zu beurteilenden Fläche 101, 102a, 102b, 102c statistisch ermittelt.

Der Bereich der Erfindung umfasst eine Messeinheit, die mittels optischer Verfahren in "Echtzeit" Schimmel in/auf Bauwerksoberflächen 101 qualitativ und quantitativ ab einem (zu bestimmenden Grenzwert) nachweist. Basis ist die Eigenschaft der meisten Schimmelarten, bei der Bestrahlung mit UV-Licht in bestimmten Wellenlängen zu fluoreszieren (Autofluoreszenz z. B. durch NADH/NADPH; Flavins; Melanine). Dieser Effekt kann mit optischen Sensoren (z. B. Kamera, Punktsensor mit streuender Linse, etc.) erfasst und mittels geeigneter Algorithmen ausgewertet werden.

Die Ergebnisse der Messungen können durch vergleichende Messungen mit UV-Beleuchtung und VIS-Beleuchtung verbessert werden. Der Sensorkopf wird für eine Messung direkt auf die zu messende Oberfläche 102 aufgesetzt, die unterschiedlichen Beleuchtungen werden nacheinander geschaltet um jeweils entsprechende Einzelaufnahmen zu generieren. Mittels eines vorher entwickelten Modells (z. B. chemometrisches Modell), können dann Art und Menge der Kontamination am Messpunkt 102 ermittelt werden.

Um die Kontamination einer Fläche 101, 102 zu bestimmen, kann eine statistische Auswertung erfolgen. Mittels eines vorher entwickelten statistischen Modells werden hierfür Messpunkte (Positionen auf der Fläche 101, 102) definiert und die jeweiligen Messwerte in das Modell übernommen. Im Ergebnis wird die Kontamination der Fläche 101, 102 ermittelt.

Die Ergebnisse für die Einzelmessung, sowie die Flächenauswertung stehen direkt zur Verfügung und können zum Beispiel mittels Bildschirm dargestellt werden.

Zur Indikation des Schimmels werden einzelne Punkte/Bereiche der Bauwerks- bzw. MaterialOberfläche 101 mit einem oder mehreren Sensoren (Punkt oder Fläche; vorzugsweise ausgebildet als spektrale Sensoren) mit verschiedenen Messbereichen (UV (ultraviolett) bis NIR (nahes Infrarot); vorzugsweise im VIS (sichtbarer Bereich)) und verschiedenen Beleuchtungen (UV bis NIR; vorzugsweise UV zur Sichtbarmachung von Fluoreszenzeffekten) aufgenommen. Die Ergebnisse werden dann mittels geeigneter Algorithmen ausgewertet. Das Ergebnis gibt unter anderem die Möglichkeit die Stärke/Menge einer Schimmelkontamination an einer entsprechenden Oberfläche / an bzw. in einem entsprechenden Material einzuschätzen. Zudem kann durch eine weiter statistische Auswertung der Reflexionsspektren (vorzugsweise im SWIR (kurzwelliges Infrarot) mittels zuvor angepasster chemometrischer Modelle die Feuchtigkeit des Baumaterials ermittelt werden. Des Weiteren können die optischen gewonnenen Messungen auch für die Beurteilung physikalischer Faktoren, zum Beispiel der Oberflächenrauhigkeit bzw. deren Änderung durch eine Vergleichsmessung, mit einem geeigneten chemometrischen Modell genutzt werden.

Obwohl manche Aspekte im Zusammenhang mit einer Vorrichtung beschrieben wurden, versteht es sich, dass diese Aspekte auch eine Beschreibung des entsprechenden Verfahrens darstellen, sodass ein Block oder ein Bauelement einer Vorrichtung auch als ein entsprechender Verfahrensschritt oder als ein Merkmal eines Verfahrensschrittes zu verstehen ist. Analog dazu stellen Aspekte, die im Zusammenhang mit einem oder als ein Verfahrensschritt beschrieben wurden, auch eine Beschreibung eines entsprechenden Blocks oder Details oder Merkmals einer entsprechenden Vorrichtung dar. Einige oder alle der Verfahrensschritte können durch einen Hardware-Apparat (oder unter Verwendung eines Hardware-Apparats), wie zum Beispiel einen Mikroprozessor, einen programmierbaren Computer oder einer elektronischen Schaltung durchgeführt werden. Bei einigen Ausführungsbeispielen können einige oder mehrere der wichtigsten Verfahrensschritte durch einen solchen Apparat ausgeführt werden.

Je nach bestimmten Implementierungsanforderungen können Ausführungsbeispiele der Erfindung in Hardware oder in Software implementiert sein. Die Implementierung kann unter Verwendung eines digitalen Speichermediums, beispielsweise einer Floppy-Disk, einer DVD, einer BluRay Disc, einer CD, eines ROM, eines PROM, eines EPROM, eines EEPROM oder eines FLASH-Speichers, einer Festplatte oder eines anderen magnetischen oder optischen Speichers durchgeführt werden, auf dem elektronisch lesbare Steuersignale gespeichert sind, die mit einem programmierbaren Computersystem derart zusammenwirken können oder zusammenwirken, dass das jeweilige Verfahren durchgeführt wird. Deshalb kann das digitale Speichermedium computerlesbar sein.

Manche Ausführungsbeispiele gemäß der Erfindung umfassen also einen Datenträger, der elektronisch lesbare Steuersignale aufweist, die in der Lage sind, mit einem programmier-baren Computersystem derart zusammenzuwirken, dass eines der hierin beschriebenen Verfahren durchgeführt wird.

Allgemein können Ausführungsbeispiele der vorliegenden Erfindung als Computerprogrammprodukt mit einem Programmcode implementiert sein, wobei der Programmcode dahin gehend wirksam ist, eines der Verfahren durchzuführen, wenn das Computerprogrammprodukt auf einem Computer abläuft.

Der Programmcode kann beispielsweise auch auf einem maschinenlesbaren Träger gespeichert sein.

Andere Ausführungsbeispiele umfassen das Computerprogramm zum Durchführen eines der hierin beschriebenen Verfahren, wobei das Computerprogramm auf einem maschinen-lesbaren Träger gespeichert ist. Mit anderen Worten ist ein Ausführungsbeispiel des erfindungsgemäßen Verfahrens somit ein Computerprogramm, das einen Programmcode zum Durchführen eines der hierin beschriebenen Verfahren aufweist, wenn das Computerprogramm auf einem Computer abläuft.

Ein weiteres Ausführungsbeispiel der erfindungsgemäßen Verfahren ist somit ein Datenträger (oder ein digitales Speichermedium oder ein computerlesbares Medium), auf dem das Computerprogramm zum Durchführen eines der hierin beschriebenen Verfahren aufgezeichnet ist.

Ein weiteres Ausführungsbeispiel des erfindungsgemäßen Verfahrens ist somit ein Datenstrom oder eine Sequenz von Signalen, der bzw. die das Computerprogramm zum Durchführen eines der hierin beschriebenen Verfahren darstellt bzw. darstellen. Der Datenstrom oder die Sequenz von Signalen kann bzw. können beispielsweise dahin gehend konfiguriert sein, über eine Datenkommunikationsverbindung, beispielsweise über das Internet, transferiert zu werden.

Ein weiteres Ausführungsbeispiel umfasst eine Verarbeitungseinrichtung, beispielsweise einen Computer oder ein programmierbares Logikbauelement, die dahin gehend konfiguriert oder angepasst ist, eines der hierin beschriebenen Verfahren durchzuführen.

Ein weiteres Ausführungsbeispiel umfasst einen Computer, auf dem das Computerprogramm zum Durchführen eines der hierin beschriebenen Verfahren installiert ist.

Ein weiteres Ausführungsbeispiel gemäß der Erfindung umfasst eine Vorrichtung oder ein System, die bzw. das ausgelegt ist, um ein Computerprogramm zur Durchführung zumindest eines der hierin beschriebenen Verfahren zu einem Empfänger zu übertragen. Die Übertragung kann beispielsweise elektronisch oder optisch erfolgen. Der Empfänger kann beispielsweise ein Computer, ein Mobilgerät, ein Speichergerät oder eine ähnliche Vorrichtung sein. Die Vorrichtung oder das System kann beispielsweise einen Datei-Server zur Übertragung des Computerprogramms zu dem Empfänger umfassen.

Bei manchen Ausführungsbeispielen kann ein programmierbares Logikbauelement (beispielsweise ein feldprogrammierbares Gatterarray, ein FPGA) dazu verwendet werden, manche oder alle Funktionalitäten der hierin beschriebenen Verfahren durchzuführen. Bei manchen Ausführungsbeispielen kann ein feldprogrammierbares Gatterarray mit einem Mikroprozessor zusammenwirken, um eines der hierin beschriebenen Verfahren durchzuführen. Allgemein werden die Verfahren bei einigen Ausführungsbeispielen seitens einer beliebigen Hardwarevorrichtung durchgeführt. Diese kann eine universell einsetzbare Hardware wie ein Computerprozessor (CPU) sein oder für das Verfahren spezifische Hardware, wie beispielsweise ein ASIC.

Die oben beschriebenen Ausführungsbeispiele stellen lediglich eine Veranschaulichung der Prinzipien der vorliegenden Erfindung dar. Es versteht sich, dass Modifikationen und Variationen der hierin beschriebenen Anordnungen und Einzelheiten anderen Fachleuten einleuchten werden. Deshalb ist beabsichtigt, dass die Erfindung lediglich durch den Schutzumfang der nachstehenden Patentansprüche und nicht durch die spezifischen Einzelheiten, die anhand der Beschreibung und der Erläuterung der Ausführungsbeispiele hierin präsentiert wurden, beschränkt sei.

## Patentansprüche

1. Vorrichtung (100, 500) zum Erfassen und Bestimmen einer Menge von unter Bestrahlung mit ultraviolettem Licht (104, 504) fluoreszierendem Schimmel auf Baustoffoberflächen (101, 501) vor Ort, aufweisend:
eine erste Lichtquelle (103, 503), die ausgebildet ist, um ultraviolettes Licht (104, 504) auf einen zu untersuchenden Bereich (102, 502) der Baustoffoberfläche (101, 501) auszusenden,
eine zweite Lichtquelle (512), die ausgebildet ist, um Licht in einem anderen Wellenlängenbereich im Vergleich zu der ersten Lichtquelle (103, 503) zu emittieren und auf den zu untersuchenden Bereich (102, 502) der Baustoffoberfläche (101, 501) auszusenden,
wobei beide Lichtquellen (503, 512) sequentiell schaltbar sind,
einen Lichtempfänger (105, 505), der ausgebildet ist, um Licht (106, 506) von dem zu untersuchenden Bereich (102, 502) zu empfangen, um Aufnahmen des zu untersuchenden Bereichs (102,502) in unterschiedlichen Beleuchtungsszenarien zu machen,
eine Steuereinheit (107, 507), die ausgebildet ist, um eine Aufnahme des mit der ersten Lichtquelle (503) beleuchteten zu untersuchenden Bereichs (102, 502) mit einer Aufnahme des mit der zweiten Lichtquelle (512) beleuchteten zu untersuchenden Bereichs (102, 502) zu vergleichen, um das mit dem Lichtempfänger (105, 505) empfangene Licht (106, 506) dahingehend zu überprüfen, ob das empfangene Licht (106, 506) zumindest anteilig fluoresziertes Licht aufweist, um qualitativ das Vorhandensein von Schimmel zu bestimmen und
wobei die Steuereinheit (107, 507) ferner ausgebildet ist, um, in dem Fall, dass ein fluoreszierender Lichtanteil erkannt wurde, die Schimmelmenge auf zumindest einem Teil der Baustoffoberfläche (101, 501), auf Grundlage einer zuvor in mindestens einem zu untersuchenden Bereich (102, 502) der Baustoffoberfläche (101, 501) ausgeführten qualitativen Erfassung von Schimmel, quantitativ zu bestimmen.

2. Vorrichtung (100, 500) nach Anspruch 1, wobei die Vorrichtung (100, 500) für eine Messung direkt auf die Baustoffoberfläche (101, 501) aufsetzbar ist, und/oder
wobei die Vorrichtung (100, 500) eine Kuppel (513) aufweist, und die Vorrichtung (100, 500) mit dem der Baustoffoberfläche (101, 501) zugewandten Ende der Kuppel (513) auf der Baustoffoberfläche (101, 501) aufsetzbar ist, und
wobei die Kuppel (513) lichtundurchlässig ausgebildet ist und/oder wobei die Kuppel (513) das der Baustoffoberfläche (101, 501) zugewandte Ende der Vorrichtung (100, 500) lichtdicht umschließt.

3. Vorrichtung (100, 500) nach einem der vorhergehenden Ansprüche, wobei die Vorrichtung (100, 500) eine spektral auflösende Optik (108, 508) aufweist, die ausgebildet ist, um das empfangene Licht (106, 506) in sein Spektrum (400A, 400B, 400C) zu zerlegen, und wobei die Steuereinheit (107, 507) ausgebildet ist, um dieses Spektrum (400A, 400B, 400C) dahingehend zu überprüfen, ob das empfangene Licht (106, 506) zumindest anteilig von Schimmel fluoresziertes Licht aufweist.

4. Vorrichtung (100, 500) nach einem der vorhergehenden Ansprüche, wobei der Lichtempfänger (505) als ein Punktsensor ausgebildet ist, wobei der Punktsensor (505) ausgebildet ist, um einen Bildpunkt innerhalb des zu untersuchenden Bereichs (102, 502) zu bestimmen und wobei die spektral auflösende Optik (108, 508) ausgebildet ist, um das in diesem Bildpunkt erfassbare Licht (106, 506) in sein Spektrum (400A, 400B, 400C) zu zerlegen, oder
wobei der Lichtempfänger (505) als ein Flächensensor ausgebildet ist, wobei der Flächensensor (505) ausgebildet ist, um einen Bildbereich zwischen 2x2 und mehr Bildpunkten innerhalb des zu untersuchenden Bereichs (102, 502) zu bestimmen und wobei die spektral auflösende Optik (108, 508) ausgebildet ist, um das in diesem Bildbereich erfassbare Licht (106, 506) in sein Spektrum (400A, 400B, 400C) zu zerlegen.

5. Vorrichtung (100, 500) nach einem der vorhergehenden Ansprüche, wobei die Steuereinheit (107, 507) ausgebildet ist, um eine Absorption und/oder Reflexion bestimmter Wellenlängenbereiche in dem empfangenen Licht (106, 506) des zu untersuchenden Bereichs (102, 502) zu erfassen und diese absorbierten und/oder reflektierten Wellenlängenbereiche als Identifikationsmerkmal zur Bestimmung der Schimmelart heranzuziehen.

6. Vorrichtung (100, 500) nach einem der vorhergehenden Ansprüche, wobei die Lichtquelle (103, 503) eine spektral breitbandige Beleuchtung aufweist, deren Spektrum den Messbereich der spektral auflösenden Optik (108, 508) überdeckt.

7. Vorrichtung (100, 500) nach einem der vorhergehenden Ansprüche, wobei mindestens eine der beiden Lichtquellen (503, 512) eine homogene Lichtquelle zur gleichmäßigen Beleuchtung des zu untersuchenden Bereichs (102, 502) ist.

8. Vorrichtung (100, 500) nach einem der vorhergehenden Ansprüche, wobei die Steuereinheit (107, 507) ausgebildet ist, um für eine stichprobenartige Bestimmung der Schimmelmenge eines Teils der Baustoffoberfläche (101, 501), die Baustoffoberfläche (101, 501) in eine Anzahl zu untersuchender Bereiche (102a, 102b, 102c) zu unterteilen, wobei die Anzahl zu untersuchender Bereiche (102a, 102b, 102c) sowie eine Anzahl in den jeweiligen zu untersuchenden Bereichen (102a, 102b, 102c) durchzuführender Stichproben basierend auf bekannten Hauptansiedlungspunkten (601, 602, 603) des Schimmels und/oder bekannten Ausbreitungscharakteristiken des Schimmels bestimmt ist.

9. Vorrichtung (100, 500) nach Anspruch 8, wobei die Steuereinheit (107, 507) ausgebildet ist, um für eine statistische Bestimmung der Schimmelmenge der Baustoffoberfläche (101, 501) die innerhalb der bestimmten Anzahl von zu untersuchenden Bereichen (102a, 102b, 102c) zuvor erfasste Schimmelmenge zu extrapolieren, um die Schimmelmenge statistisch auf die Baustoffoberfläche (101, 501) hochzurechnen.

10. Verfahren (700) zum Erfassen und Bestimmen einer Menge von unter Bestrahlung mit ultraviolettem Licht (104, 504) fluoreszierendem Schimmel auf Baustoffoberflächen (101, 501) vor Ort, mit den folgenden Schritten:
Bereitstellen einer ersten Lichtquelle (103, 503) zum Aussenden (701) von ultraviolettem Licht (104, 504) auf einen zu untersuchenden Bereich (102, 502) der Baustoffoberfläche (101, 501),
Bereitstellen einer zweiten Lichtquelle (512) zum Aussenden von Licht in einem im Vergleich zu der ersten Lichtquelle (103, 503) verschiedenen Wellenlängenbereich,
sequentielles Schalten der ersten und der zweiten Lichtquelle (503, 512),
Empfangen (702) von Licht (106, 506) von dem zu untersuchenden Bereich (102, 502),
Erstellen einer Aufnahme des mit der ersten Lichtquelle (103, 503) beleuchteten zu untersuchenden Bereichs (102, 502),
Erstellen einer Aufnahme des mit der zweiten Lichtquelle (512) beleuchteten zu untersuchenden Bereichs (102, 502), und
Vergleichen der beiden Aufnahmen miteinander, beinhaltend ein
Überprüfen (703), ob das empfangene Licht (106, 506) zumindest anteilig fluoresziertes Licht aufweist, und
falls ein fluoreszierender Lichtanteil erkannt wurde (704), quantitatives Bestimmen (705) der Schimmelmenge auf zumindest einem Teil der Baustoffoberfläche (101, 501) auf Grundlage einer zuvor in zumindest einem zu untersuchenden Bereich (102, 502) der Baustoffoberfläche (101, 501) ausgeführten qualitativen Erfassung von Schimmel.

11. Verfahren (700) nach Anspruch 10, wobei das Verfahren direkt auf der Baustoffoberfläche ausgeführt wird, und/oder wobei das Verfahren (700) zerstörungsfrei für den zu untersuchenden Bereich (102, 502) und für die Baustoffoberfläche (101, 501) selbst durchführbar ist.

12. Verfahren (700) nach Anspruch 10 oder 11, wobei das Verfahren (700) beinhaltet, vor dem Überprüfen (703), ob das empfangene Licht (106, 506) zumindest anteilig von Schimmel fluoresziertes Licht aufweist, das empfangene Licht (106, 506) in dessen Spektrum (400A, 400B, 400C) zu zerlegen.

13. Verfahren (700) nach einem der Ansprüche 10 bis 12, ferner aufweisend:
Erfassen einer Absorption bestimmter Wellenlängenbereiche in dem empfangenen Licht (106, 506) des zu untersuchenden Bereichs (102, 502), und
Heranziehen dieser absorbierten Wellenlängenbereiche als Identifikationsmerkmal zur Bestimmung der Schimmelart.

14. Verfahren (700) nach einem der Ansprüche 10 bis 13, wobei der Schritt des quantitativen Bestimmens (705) der Schimmelmenge auf zumindest einem Teil der Baustoffoberfläche (101, 501) eine stichprobenartige Bestimmung umfasst, und das Verfahren ferner den folgenden Schritt umfasst:
Unterteilen der Baustoffoberfläche (101, 501) in eine bestimmte Anzahl zu untersuchender Bereiche (102a, 102b, 102c), wobei die Anzahl zu untersuchender Bereiche (102a, 102b, 102c) sowie eine Anzahl in den jeweiligen zu untersuchenden Bereichen (102a, 102b, 102c) durchzuführender Stichproben basierend auf bekannten Hauptansiedlungspunkten (601, 602, 603) des Schimmels und/oder bekannten Ausbreitungscharakteristiken des Schimmels bestimmt wird.

15. Verfahren (700) nach einem der Ansprüche 10 bis 14, wobei das Verfahren (700) ferner einen Schritt zum quantitativen Bestimmen der Schimmelmenge der gesamten Baustoffoberfläche (101, 501) mittels einer statistischen Bestimmung umfasst, wobei die statistische Bestimmung umfasst:
Extrapolieren der innerhalb der bestimmten Anzahl von zu untersuchenden Bereichen (102a, 102b, 102c) zuvor quantitativ bestimmten Schimmelmenge, um die Schimmelmenge statistisch auf die gesamte Baustoffoberfläche (101, 501) hochzurechnen.

## Claims

1. Device (100, 500) for sensing and determining an amount of mold on building-material surfaces (101, 501) in the field, said mold being fluorescent when irradiated with ultraviolet light (104, 504), comprising:
a first light source (103, 503) configured to radiate ultraviolet light (104, 504) onto an area to be examined (102, 502) of the building-material surface (101, 501),
a second light source (512) configured to emit light within a wavelength range that is different as compared to that of the first light source (103, 503) and to radiate same onto that area to be examined (102, 502) of the building-material surface (101, 501),
both light sources (503, 512) being sequentially switchable,
a light receiver (105, 505) configured to receive light (106, 506) from the area to be examined (102, 502) so as to take pictures of the area to be examined (102, 502) in different illumination scenarios,
a control unit (107, 507) configured to compare a picture taken of the area to be examined (102, 502) that is illuminated with the first light source (503) to a picture taken of the area to be examined (102, 502) which is illuminated with the second light source (512), so as to inspect the light (106, 506) received by means of the light receiver (105, 505) in terms of whether the light (106, 506) received comprises light that has fluoresced at least in portions so as to at least qualitatively determine the presence of mold, and
wherein the control unit (107, 507) is further configured to quantitatively determine, in the event that a fluorescent portion of light has been identified, the amount of mold present on at least part of the building-material surface (101, 501) on the basis of qualitative sensing of mold that was previously performed in at least an area to be examined (102, 502) of the building-material surface (101, 501).

2. Device (100, 500) as claimed in claim 1, wherein the device (100, 500) may be directly placed onto the building-material surface (101, 501) for measurement purposes, and/or
wherein the device (100, 500) comprises a dome (513), and the device (100, 500) can be placed onto the building-material surface (101, 501) with that end of the dome (513) which faces the building-material surface (101, 501), and
wherein the dome (513) is configured to be optically opaque and/or wherein the dome (513) encloses that end of the device (100, 500) in a light-tight manner which faces the building-material surface (101, 501).

3. Device (100, 500) as claimed in any of the previous claims, the device (100, 500) comprising a spectrally resolving optic (108, 508) configured to decompose the light (106, 506) received into its spectrum (400A, 400B, 400C), and wherein the control unit (107, 507) is configured to inspect said spectrum (400A, 400B, 400C) in terms of whether the light (106, 506) received comprises, at least in portions, light that is fluoresced by mold.

4. Device (100, 500) as claimed in any of the previous claims, wherein the light receiver (505) is configured as a point sensor, the point sensor (505) being configured to determine a pixel within the area to be examined (102, 502), and wherein the spectrally resolving optic (108, 508) is configured to decompose the light (106, 506), which can be sensed in this pixel, into its spectrum (400A, 400B, 400C), or
wherein the light receiver (505) is configured as an area sensor, said area sensor (505) being configured to determine an image area of between 2x2 and more pixels within the area to be examined (102, 502), and wherein the spectrally resolving optic (108, 508) is configured to decompose the light (106, 506), which can be sensed in this image area, into its spectrum (400A, 400B, 400C).

5. Device (100, 500) as claimed in any of the previous claims, wherein the control unit (107, 507) is configured to sense absorption and/or reflection of specific wavelength ranges within the light (106, 506) received of the area to be examined (102, 502) and to use said absorbed and/or reflected wavelength ranges as an identification feature for determining the type of mold.

6. Device (100, 500) as claimed in any of the previous claims, wherein the light source (103, 503) comprises spectrally broad-band illumination, the spectrum of which overlays the range of measurement of the spectrally resolving optic (108, 508).

7. Device (100, 500) as claimed in any of the previous claims, wherein at least one of the two light sources (503, 512) is a homogenous light source for uniform illumination of the area to be examined (102, 502).

8. Device (100, 500) as claimed in any of the previous claims, wherein the control unit (107, 507) is configured to subdivide the building-material surface (101, 501) into a number of areas (102a, 102b, 102c) to be examined for the purpose of determining the amount of mold of part of the building-material surface (101, 501) on the basis of random samples, wherein the number of areas (102a, 102b, 102c) to be examined as well as a number of random samples to be taken in the areas (102a, 102b, 102c) to be examined in each case are determined on the basis of the known main points of occurrence (601, 602, 603) of the mold and/or known propagation characteristics of the mold.

9. Device (100, 500) as claimed claim 8, wherein the control unit (107, 507) is configured to extrapolate, for the purpose of statistically determining the amount of mold of the building-material surface (101, 501), the amount of mold previously sensed within the determined number of areas (102a, 102b, 102c) to be examined so as to make a statistic projection of the amount of mold to the building-material surface (101, 501).

10. Method (700) of sensing and determining an amount of mold on building-material surfaces (101, 501) in the field, said mold being fluorescent when irradiated with ultraviolet light (104, 504), comprising the following steps:
providing a first light source (103, 503) for radiating (701) ultraviolet light (104, 504) onto an area to be examined (102, 502) of the building-material surface (101, 501), providing a second light source (512) for radiating light within a wavelength range that is different as compared to that of the first light source (103, 503),
sequentially switching the first and second light sources (503, 512),
receiving (702) light (106, 506) from the area to be examined (102, 502),
taking a picture of the area to be examined (102, 502) that is illuminated by the first light source (103, 503),
taking a picture of the area to be examined (102, 502) that is illuminated by the second light source (512), and
comparing the two pictures with each other, including
inspecting (703) whether the light (106, 506) received comprises light that has fluoresced at least in portions, and
in the event that a fluorescent portion of light has been identified (704), quantitatively determining (705) the amount of mold present on at least part of the building-material surface (101, 501) on the basis of qualitative sensing of mold that was previously performed in at least an area to be examined (102, 502) of the building area surface (101, 501).

11. Method (700) as claimed in claim 10, the method being performed directly on the building-material surface, and/or the method (700) being able to be performed in a non-destructive manner for the area to be examined (102, 502) and for the building-material surface (101, 501) itself.

12. Method (700) as claimed in claims 10 or 11, the method (700) comprising, prior to inspecting (703) whether the light (106, 506) received comprises, at least in portions, light that is fluoresced by mold, decomposing the light (106, 506) received into its spectrum (400A, 400B, 400C).

13. Method (700) as claimed in any of claims 10 to 12, further comprising:
sensing absorption of specific wavelength ranges within the light (106, 506) received of the area to be examined (102, 502), and
using said absorbed wavelength ranges as an identification feature for determining the type of mold.

14. Method (700) as claimed in any of claims 10 to 13, wherein the step of quantitatively determining (705) the amount of mold present on at least part of the building-material surface (101, 501) includes determination on the basis of random samples, said method further comprising the following step:
subdividing the building-material surface (101, 501) into a specific number of areas (102a, 102b, 102c) to be examined, wherein the number of areas to be examined (102a, 102b, 102c) as well as a number of random samples to be taken in the areas to be examined (102a, 102b, 102c) in each case are determined on the basis of the known main points of occurrence (601, 602, 603) of the mold and/or known propagation characteristics of the mold.

15. Method (700) as claimed in any of claims 10 to 14, said method (700) further comprising a step of quantitatively determining the amount of mold of the entire building-material surface (101, 501) by means of statistic determination, said statistic determination including:
extrapolating the amount of mold that was previously quantitatively determined within the determined number of areas to be examined (102a, 102b, 102c) so as to so as to make a statistic projection of the amount of mold to the entire building-material surface (101, 501).

## Revendications

1. Dispositif (100, 500) pour détecter et déterminer sur le site une quantité de moisissure fluorescente sous irradiation par de la lumière ultraviolette (104, 504) sur des surfaces d'un matériau de construction (101, 501), présentant:
une première source de lumière (103, 503) qui est conçue pour émettre de la lumière ultraviolette (104, 504) vers une zone à examiner (102, 502) de la surface du matériau de construction (101, 501),
une deuxième source de lumière (512) qui est conçue pour émettre de la lumière dans une plage de longueurs d'onde différente en comparaison avec la première source de lumière (103, 503) et pour l'émettre vers la zone à examiner (102, 502) de la surface du matériau de construction (101, 501),
dans lequel les deux sources de lumière (503, 512) peuvent être commutées en séquence,
un récepteur de lumière (105, 505) qui est conçu pour recevoir de la lumière (106, 506) de la zone à examiner (102, 502) pour réaliser des photographies de la zone à examiner (102, 502) dans différents scénarios d'éclairage,
une unité de commande (107, 507) qui est conçue pour comparer une photographie de la zone à examiner (102, 502) éclairée par la première source de lumière (503) avec une photographie de la zone à examiner (102, 502) éclairée par la deuxième source de lumière (512) pour examiner la lumière reçue (106, 506) par le récepteur de lumière (105, 505) pour savoir si la lumière reçue (106, 506) présente de la lumière au moins proportionnellement fluorescente pour déterminer qualitativement la présence de moisissure et,
dans lequel l'unité de commande (107, 507) est par ailleurs conçue pour déterminer quantitativement, au cas où a été détectée une part de lumière fluorescente, la quantité de moisissure sur au moins une partie de la surface du matériau de construction (101, 501), sur base d'une détection qualitative de moisissure réalisée auparavant dans au moins une zone à examiner (102, 502) de la surface du matériau de construction (101, 501).

2. Dispositif (100, 500) selon la revendication 1, dans lequel le dispositif (100, 500) peut, pour une mesure, être placé directement sur la surface du matériau de construction (101, 501), et/ou
dans lequel le dispositif (100, 500) présente un dôme (513), et le dispositif (100, 500) peut être placé, par l'extrémité du dôme (513) opposée à la surface du matériau de construction (101, 501), sur la surface du matériau de construction (101, 501), et
dans lequel le dôme (513) est réalisé opaque et/ou dans lequel le dôme (513) entoure l'extrémité du dispositif (100, 500) orientée vers la surface du matériau de construction (101, 501) de manière opaque.

3. Dispositif (100, 500) selon l'une des revendications précédentes, dans lequel le dispositif (100, 500) présente une optique à résolution spectrale (108, 508) qui est conçue pour décomposer la lumière reçue (106, 506) en son spectre (400A, 400B, 400C), et dans lequel l'unité de commande (107, 507) est conçue pour vérifier ce spectre (400A, 400B, 400C) pour savoir si la lumière reçue (106, 506) présente de la lumière au moins proportionnellement fluorescente par suite de moisissure.

4. Dispositif (100, 500) selon l'une des revendications précédentes, dans lequel le récepteur de lumière (505) est conçu comme un capteur ponctuel, dans lequel le capteur ponctuel (505) est conçu pour déterminer un pixel dans la zone à examiner (102, 502) et dans lequel l'optique à résolution spectrale (108, 508) est conçue pour décomposer la lumière (106, 506) détectable dans ce pixel en son spectre (400A, 400B, 400C), ou
dans lequel le récepteur de lumière (505) est conçu comme capteur de surface, dans lequel le capteur de surface (505) est conçu pour déterminer une zone d'image comprise entre 2x2 et plus de pixels dans la zone à examiner (102, 502) et dans lequel l'optique à résolution spectrale (108, 508) est conçue pour décomposer la lumière (106, 506) détectable dans cette zone d'image en son spectre (400A, 400B, 400C).

5. Dispositif (100, 500) selon l'une des revendications précédentes, dans lequel l'unité de commande (107, 507) est conçue pour détecter une absorption et/ou réflexion de certaines plages de longueurs d'onde dans la lumière reçue (106, 506) de la zone à examiner (102, 502) et pour utiliser ces plages de longueurs d'onde absorbées et/ou réfléchies comme caractéristique d'identification pour déterminer le type de moisissure.

6. Dispositif (100, 500) selon l'une des revendications précédentes, dans lequel la source de lumière (103, 503) présente un éclairage spectralement de bande large dont le spectre couvre la plage de mesure de l'optique à résolution spectrale (108, 508).

7. Dispositif (100, 500) selon l'une des revendications précédentes, dans lequel au moins une des deux sources de lumière (503, 512) est une source de lumière homogène destinée à éclairer de manière uniforme la zone à examiner (102, 502).

8. Dispositif (100, 500) selon l'une quelconque des revendications précédentes, dans lequel l'unité de commande (107, 507) est conçue pour subdiviser, pour une détermination aléatoire de la quantité de moisissure d'une partie de la surface du matériau de construction (101, 501), la surface du matériau de construction (101, 501) en un nombre de zones à examiner (102a, 102b, 102c), dans lequel le nombre de zones à examiner (102a, 102b, 102c) ainsi qu'un nombre d'essais aléatoires à effectuer dans les zones à examiner (102a, 102b, 102c) respectives sur base de points d'implantation principaux connus (601, 602, 603) de la moisissure et/ou de caractéristiques de propagation connues de la moisissure.

9. Dispositif (100, 500) selon la revendication 8, dans lequel l'unité de commande (107, 507) est conçue pour extrapoler, pour une détermination statistique de la quantité de moisissure à la surface du matériau de construction (101, 501), la quantité de moisissure détectée auparavant dans le nombre déterminé de zones à examiner (102a, 102b, 102c) pour calculer statistiquement la quantité de moisissure à la surface du matériau de construction (101, 501).

10. Procédé (700) pour détecter et déterminer sur le site une quantité de moisissure fluorescente sous irradiation par de la lumière ultraviolette (104, 504) sur des surfaces d'un matériau de construction (101, 501), aux étapes suivantes consistant à:
prévoir une première source de lumière (103, 503) pour émettre (701) de la lumière ultraviolette (104, 504) vers une zone à examiner (102, 502) de la surface du matériau de construction (101, 501),
prévoir une deuxième source de lumière (512) pour émettre de la lumière dans une plage de longueurs d'onde différente en comparaison avec la première source de lumière (103, 503),
commuter en séquence la première et la deuxième source de lumière (503, 512),
recevoir (702) de la lumière (106, 506) de la zone à examiner (102, 502),
réaliser une photographie de la zone à examiner (102, 502) éclairée par la première source de lumière (103, 503),
réaliser une photographie de la zone à examiner (102, 502) éclairée par la deuxième source de lumière (512), et
comparer les deux photographies entre elles, ce qui comporte le fait de
vérifier (703) si la lumière reçue (106, 506) présente de la lumière au moins proportionnellement fluorescente, et
si une part de lumière fluorescente a été détectée (704), déterminer de manière quantitative (705) la quantité de moisissure sur au moins une partie de la surface du matériau de construction (101, 501) sur base d'une détection qualitative de moisissure effectuée auparavant dans au moins une zone à examiner (102, 502) de la surface du matériau de construction (101, 501).

11. Procédé (700) selon la revendication 10, dans lequel le procédé est réalisé directement sur la surface du matériau de construction, et/ou dans lequel le procédé (700) peut être réalisé sans destruction pour la zone à examiner (102, 502) et pour la surface du matériau de construction (101, 501) elle-même.

12. Procédé (700) selon la revendication 10 ou 11, dans lequel le procédé (700) comporte, avant de vérifier (703) si la lumière reçue (106, 506) présente de la lumière au moins proportionnellement fluorescente par suite de moisissure, le fait de décomposer la lumière reçue (106, 506) en son spectre (400A, 400B, 400C).

13. Procédé (700) selon l'une des revendications 10 à 12, présentant par ailleurs le fait de:
détecter une absorption de plages de longueurs d'onde déterminées dans la lumière reçue (106, 506) de la zone à examiner (102, 502), et
utiliser ces plages de longueurs d'onde absorbées comme caractéristique d'identification pour déterminer le type de moisissure.

14. Procédé (700) selon l'une des revendications 10 à 13, dans lequel l'étape de détermination quantitative (705) de la quantité de moisissure sur au moins une partie de la surface du matériau de construction (101, 501) comporte une détermination aléatoire, et le procédé comporte par ailleurs l'étape suivante consistant à:
subdiviser la surface du matériau de construction (101, 501) en un nombre déterminé de zones à examiner (102a, 102b, 102c), où le nombre de zones à examiner (102a, 102b, 102c) ainsi qu'un nombre d'essais aléatoires à effectuer dans les zones à examiner respectives (102a, 102b, 102c) sont déterminés sur base de points d'implantation principaux connus (601, 602, 603) de la moisissure et/ou de caractéristiques de propagation connues de la moisissure.

15. Procédé (700) selon l'une des revendications 10 à 14, dans lequel le procédé (700) comporte par ailleurs une étape de détermination quantitative de la quantité de moisissure de toute la surface du matériau de construction (101, 501) au moyen d'une détermination statistique, dans lequel la détermination statistique comporte le fait de:
extrapoler la quantité de moisissure déterminée de manière quantitative auparavant dans le nombre déterminé de zones à examiner (102a, 102b, 102c) pour calculer la quantité de moisissure de manière statistique sur toute la surface du matériau de construction (101, 501).
